# EUROPEAN PATENT APPLICATION

(11) **EP 4 530 281 A1**
(43) Date of publication of application: **02.04.2025**
(21) Application number: 24198418.6
(22) Date of filing: 04.09.2024
(51) Int. Cl.: C07C 67/08, C07C 67/29, C07C 69/16

(54) **8-(ACYLOXY)ALKANAL COMPOUND, PROCESS FOR PREPARING THE SAME AND PROCESS FOR PREPARING 2,9-DIACETOXYUNDECANE FROM THE 8-(ACYLOXY)ALKANAL COMPOUND**

(30) Priority: 07.09.2023 JP 2023145491
(71) Applicant: SHIN-ETSU CHEMICAL CO., LTD., Tokyo 100-0005 (JP)
(72) Inventor: MIYAKE, Yuki, Niigata, 942-8601 (JP); WATANABE, Takeru, Niigata, 942-8601 (JP); KOMATSU, Ryo, Niigata, 942-8601 (JP)
(74) Representative: De Vries & Metman

(57) **Abstract**

The present invention provides an 8-(acyloxy)alkanal compound (1), wherein n represents 0 or 1, and R¹ represents a linear or branched alkyl group having 1 to 10 carbon atoms, a phenyl group, or a phenyl group in which one or more hydrogen atoms are substituted with a halogen atom, and a process for preparing the same. The process comprises the steps of subjecting the aforesaid 8-(acyloxy)alkanal compound (1) to a nucleophilic addition reaction with a nucleophilic reagent, alkyl compound (2), wherein M¹ represents Li, MgZ¹, CuZ¹, or CuLiZ¹, n represents 0 or 1, and Z¹ represents a halogen atom, a methyl group, or an ethyl group, to form 2,9-undecanediol (3), and subjecting the 2,9-undecanediol (3) to an acetylation reaction to form the 2,9-diacetoxyundecane (4).

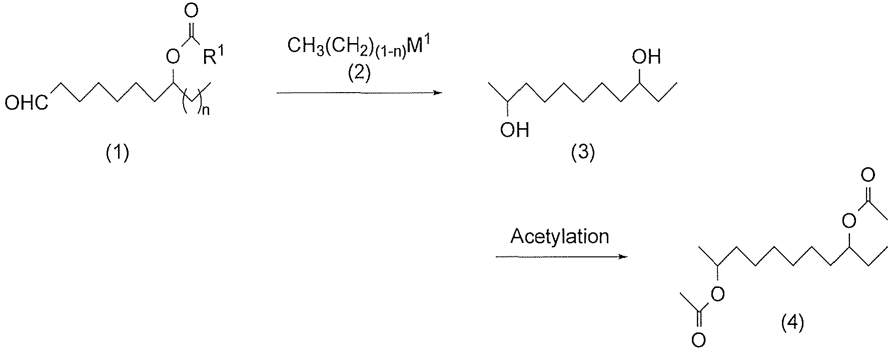

## Description

### TECHNICAL FIELD

The present invention relates to an 8-(acyloxy)alkanal compound, and a process for preparing the 8-(acyloxy)alkanal compound. The present invention also relates to a process for preparing 2,9-diacetoxyundecane from the aforesaid 8-(acyloxy)alkanal compound. The present invention also relates to processes for preparing an 8-(acyloxy)nonanal compound, an 8-(acyloxy)decanal compound, and 2,9-diacetoxyundecane from the aforesaid 8-(acyloxy)alkanal compound.

### BACKGROUND ART

The Swede midge (scientific name: *Contarinia nasturtii*) is a vegetable pest, native to Europe, that feeds on plants of the Brassica family, such as broccoli and cauliflower. Swede midge larvae feed on the leaves and flowers of the plants by secreting digestive juices that break down the cuticle, resulting in twisted, rounded, and wrinkled leaves, stringy leaf damage, and engorged leaf stalks which markedly reduce the product value. Because the larvae are protected by the leaves, typical pesticides are often ineffective due to poor contact, making control difficult. Biological control methods are attracting attention due to concerns about residual pesticides, and one promising method is the use of sex pheromones (Non-Patent Literature 1 below).

The sex pheromone composition of the Swede midge is reported to be a 1:2:0.02 mixture of (2S, 95)-2,9-diacetoxyundecane, (2S, 1 05)-2,10-diacetoxyundecane, and (2S)-2-acetoxyundecane (Non-Patent Literature 2 below).

The following process, for example, has been reported as a process for preparing the aforesaid (2S, 9S)-2,9-diacetoxyundecane, and includes a total of seven steps with a yield of 21.39% (Non-Patent Literature 2 below). The process includes reacting 7-bromo-1-heptene, a starting material, with magnesium in tetrahydrofuran (THF) to form 6-heptenylmagnesium bromide, and then subjecting the 6-heptenylmagnesium bromide to a nucleophilic addition reaction with propylene oxide in the presence of a copper catalyst to form (2*S*)-9-decen-2-ol. (2*S*)-9-Decen-2-ol thus obtained is then reacted with sodium hydride in tetrahydrofuran in the presence of tetrabutylammonium iodide and then reacted with benzyl bromide to form (2*S*)-2-benzyloxy-9-decene. (2*S*)-2-Benzyloxy-9-decene thus obtained is then reacted with AD-mix-β in hydrous *tert*-butyl alcohol to form (2*R*, 9*S*)-9-(benzyloxy)-1,2-decanediol. (2*R*, 9S)-9-(Benzyloxy)-1,2-decanediol thus obtained is then reacted with p-toluenesulfonyl chloride in pyridine to form (2*R*, 9*S*)-1-(4-methylbenzenesulfonate)-9-(benzyloxy)-1,2-decanediol. (2*R*, 9*S*)-1-(4-Methylbenzenesulfonate)-9-(benzyloxy)-1,2-decanediol thus obtained is then reacted with methylmagnesium chloride in tetrahydrofuran to form (3S, 10S)-10-(benzyloxy)-3-undecanol, followed by reacting (3*S*, 10*S*)-10-(benzyloxy)-3-undecanol thus obtained with hydrogen in ethanol in the presence of a palladium on carbon catalyst to form (2*S*, 9*S*)-2,9-undecanediol. (2*S*, 9*5*)-2,9-Undecanediol thus obtained is acetylated with acetic anhydride in the presence of pyridine and 4-dimethylaminopyridine (DMAP) to obtain the aforesaid (2*S*, 9*S*)-2,9-diacetoxyundecane, the target compound.

### PRIOR ART

### [Patent Literature]

[Patent Literature 1] Japanese Patent Application Laid-Open No. 2019-189535.

### [Non-Patent Literatures]

[Non-Patent Literature 1] Elisabeth A. Hodgdon et al., The Canadian Entomologist, 2022, 154, e37, 1-17.
[Non-Patent Literature 2] Ylva Hillbur et al., J. Chem. Ecol., 2005, 31 (8), 1807-1828.

### OBJECT OF THE INVENTION

However, the preparation process of Non-Patent Literature 2 uses highly toxic and expensive AD-mix-β in five times the amount of the starting material, making the process unsuited to industrial applications. Here, AD-mix-β is a mixture of hydroquinidine-1,4-phthalazinediyl diether which is expensive, potassium carbonate, and potassium ferricyanide, and potassium osmate dihydrate which is highly toxic. The preparation process of Non-Patent Literature 2 also uses ignitable palladium on carbon, making the process unsuited to industrial applications. Industrialization of the preparation process of Non-Patent Literature 2 also is difficult due to the large number of steps as well as low yield.

The present invention has been made in view of the aforementioned circumstances, and aims to provide a novel synthetic intermediate useful for preparing 2,9-diacetoxyundecane, and a process for preparing the synthetic intermediate. The present invention also aims to provide an efficient process for preparing 2,9-diacetoxyundecane from the aforesaid synthetic intermediate, preferably an efficient and economic process for preparing 2,9-diacetoxyundecane from the aforesaid synthetic intermediate, and particularly preferably a process for preparing 2,9-diacetoxyundecane from the aforesaid synthetic intermediate that is suited to industrial applications.

### SUMMARY OF THE INVENTION

As a result of intensive research to overcome the aforesaid problems of the prior art, the present inventors have found that a key intermediate that can synthesize 2,9-diacetoxyundecane is a novel compound, 8-(acyloxy)alkanal compound. The present inventors have also found a process, particularly an inexpensive and efficient process, for preparing the novel compound, 8-(acyloxy)alkanal compound, and found a process, particularly an efficient process, for preparing 2,9-diacetoxyundecane, the sex pheromone of the Swede midge, from the 8-(acyloxy)alkanal compound, and thus have completed the present invention. The aforesaid process for preparing the aforesaid 8-(acyloxy)alkanal compound and the aforesaid process for preparing the aforesaid 2,9-diacetoxyundecane also have been found to be suited to industrial applications.

According to a first aspect of the present invention, there is provided an 8-(acyloxy)alkanal compound of the following general formula (1): 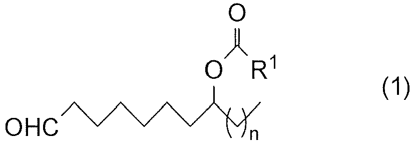 wherein n represents 0 or 1, and R¹ represents a linear or branched alkyl group having 1 to 10 carbon atoms, a phenyl group, or a phenyl group in which one or more hydrogen atoms are substituted with a halogen atom.

According to a second aspect of the present invention, there is provided a process for preparing 2,9-diacetoxyundecane of the following formula (4): 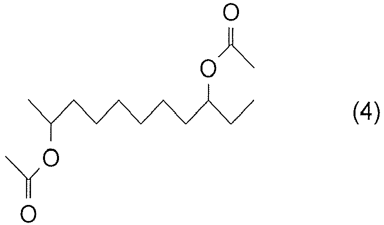 the process comprising the steps of subjecting an 8-(acyloxy)alkanal compound of the following general formula (1): 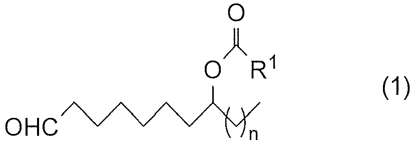 wherein n represents 0 or 1, and R¹ represents a linear or branched alkyl group having 1 to 10 carbon atoms, a phenyl group, or a phenyl group in which one or more hydrogen atoms are substituted with a halogen atom, to a nucleophilic addition reaction with a nucleophilic reagent, alkyl compound, of the following general formula (2):

CH₃(CH₂)_{(1-*n*})M¹ (2)

wherein M¹ represents Li, MgZ¹, CuZ¹, or CuLiZ¹, n represents 0 or 1, and Z¹ represents a halogen atom, a methyl group, or an ethyl group,
to form 2,9-undecanediol of the following formula (3): 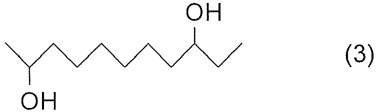 and subjecting the 2,9-undecanediol (3) to an acetylation reaction to form the aforesaid 2,9-diacetoxyundecane (4).

According to a third aspect of the present invention, there is provided a process for preparing an 8-(acyloxy)alkanal compound of the following general formula (1): 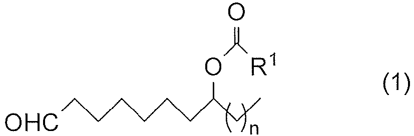 wherein n represents 0 or 1, and R¹ represents a linear or branched alkyl group having 1 to 10 carbon atoms, a phenyl group, or a phenyl group in which one or more hydrogen atoms are substituted with a halogen atom,
the process comprising steps of
subjecting a dialkoxyacyloxyalkane compound of the following general formula (5): 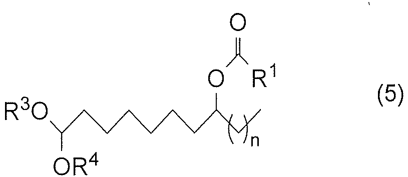 wherein n represents 0 or 1, R¹ is as defined above, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
to a hydrolysis reaction to form the aforesaid 8-(acyloxy)alkanal compound (1).

According to a fourth aspect of the present invention, there is provided a process for preparing the aforesaid 8-(acyloxy)alkanal compound (1) as defined in the third aspect, with proviso that *n* is 0 (in the present specification, also referred to as "8-(acyloxy)nonanal compound (1: *n* = 0)" or "8-(acyloxy)nonanal compound (1), wherein n = 0"),
the process comprising the steps of
converting a 6-halo-1,1-dialkoxyhexane compound of the following general formula (6): 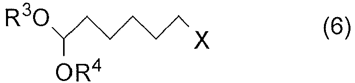 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
into a nucleophilic reagent, 6,6-dialkoxyhexyl compound, of the following general formula (7): 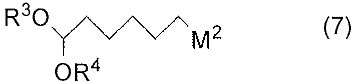 wherein M² represents Li, MgZ², CuZ², or CuLiZ², Z² represents a halogen atom or a 6,6-dialkoxyhexyl group, and R³ and R⁴ are as defined above, subsequently subjecting the nucleophilic reagent, 6,6-dialkoxyhexyl compound (7), to a nucleophilic addition reaction with propylene oxide of the following formula (8): 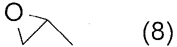 to form a 9,9-dialkoxy-2-nonanol compound of the following general formula (9A): 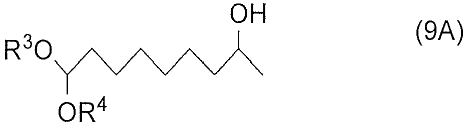 wherein R³ and R⁴ are as defined above,
subjecting the 9,9-dialkoxy-2-nonanol compound (9A) to acylation to form a 9,9-dialkoxy-2-acyloxynonane compound of the following general formula (5A), which is the general formula (5) above, wherein n is 0: 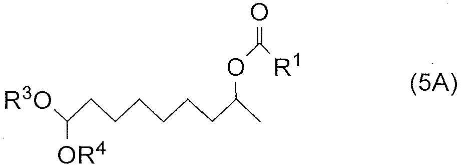 wherein R¹, R³, and R⁴ are as defined above,
and the aforesaid preparation process according to the third aspect, comprising subjecting the aforesaid 9,9-dialkoxy-2-acyloxynonane compound (5A) to a hydrolysis reaction to form the aforesaid 8-(acyloxy)nonanal compound (1), wherein n = 0.

According to a fifth aspect of the present invention, there is provided a process for preparing the aforesaid 8-(acyloxy)alkanal compound (1) as defined in the third aspect, with proviso that n is 1 (in the present specification, also referred to as "8-(acyloxy)decanal compound (1: *n* = 1)" or "8-(acyloxy)decanal compound (1), wherein n = 1"),
the process comprising the steps of
converting a 6-halo-1,1-dialkoxyhexane compound of the following general formula (6): 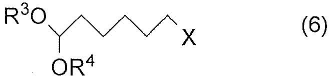 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms, into a nucleophilic reagent, 6,6-dialkoxyhexyl compound, of the following general formula (7): 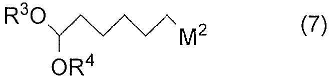 wherein M² represents Li, MgZ², CuZ², or CuLiZ², Z² represents a halogen atom or a 6,6-dialkoxyhexyl group, and R³ and R⁴ are as defined above, subsequently subjecting the nucleophilic reagent, 6,6-dialkoxyhexyl compound (7), to a nucleophilic addition reaction with 1,2-butylene oxide of the following formula (10): 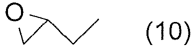 to form a 10,10-dialkoxy-3-decanol compound of the following general formula (9B): 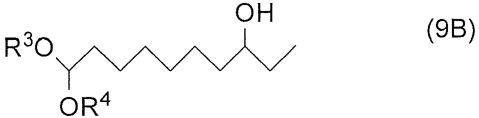 wherein R³ and R⁴ are as defined above, subjecting the 10,10-dialkoxy-3-decanol (9B) to acylation to form a 10,10-dialkoxy-3-acyloxydecane compound of the following general formula (5B), which is the general formula (5) above, wherein n is 1: 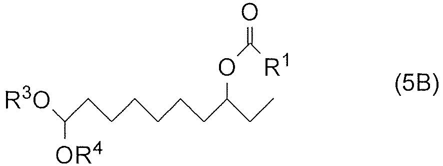 wherein R¹, R³, and R⁴ are as defined above,
and the aforesaid preparation process according to the third aspect, comprising subjecting the aforesaid 10,10-dialkoxy-3-acyloxydecane compound (5B) to a hydrolysis reaction to form the aforesaid 8-(acyloxy)decanal compound (1), wherein n = 1.

According to a sixth aspect of the present invention, there is provided a process for preparing 2,9-diacetoxyundecane (4) as defined in the second aspect, the process comprising the steps of
the aforesaid preparation process according to the third aspect, comprising preparing the aforesaid 8-(acyloxy)alkanal compound (1) from the aforesaid dialkoxyacyloxyalkane compound (5), and
and the aforesaid preparation process according to the second aspect, comprising preparing the aforesaid 2,9-diacetoxyundecane (4) from the aforesaid 8-(acyloxy)alkanal compound (1).

According to a seventh aspect of the present invention, there is provided the process for preparing 2,9-diacetoxyundecane (4) as defined in the second aspect, the process comprising the steps of
converting a 6-halo-1, 1-dialkoxyhexane compound of the following general formula (6): 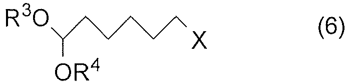 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
into a nucleophilic reagent, 6,6-dialkoxyhexyl compound, of the following general formula (7): 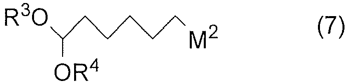 wherein M² represents Li, MgZ², CuZ², or CuLiZ², Z² represents a halogen atom or a 6,6-dialkoxyhexyl group, and R³ and R⁴ are as defined above,
subsequently subjecting the nucleophilic reagent, 6,6-dialkoxyhexyl compound (7), to a nucleophilic addition reaction with propylene oxide of the following formula (8): 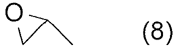 to form a 9,9-dialkoxy-2-nonanol compound of the following general formula (9A): 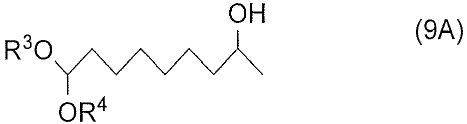 wherein R³ and R⁴ are as defined above,
subjecting the 9,9-dialkoxy-2-nonanol compound (9A) to acylation to form a 9,9-dialkoxy-2-acyloxynonane compound of the following general formula (5A), which is the general formula (5) above, wherein *n* is 0: 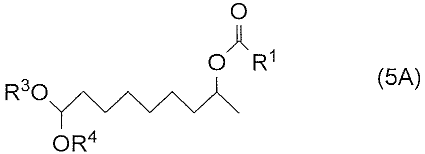 wherein R¹, R³, and R⁴ are as defined above,
subjecting the 9,9-dialkoxy-2-acyloxynonane compound (5A) to a hydrolysis reaction to form an 8-(acyloxy)alkanal compound (1), wherein n = 0, which is the general formula (1) above, wherein n is 0,
and the aforesaid preparation process according to the second aspect, comprising preparing the 2,9-diacetoxyundecane (4) from the 8-(acyloxy)alkanal compound (1), wherein n = 0.

According to an eighth aspect of the present invention, there is provided a process for preparing 2,9-diacetoxyundecane (4) as defined in the second aspect, the process comprising the steps of
converting a 6-halo-1,1-dialkoxyhexane compound of the following general formula (6): 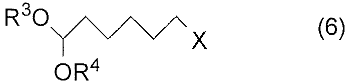 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms, into a nucleophilic reagent, 6,6-dialkoxyhexyl compound, of the following general formula (7): 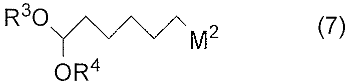 wherein M² represents Li, MgZ², CuZ², or CuLiZ², Z² represents a halogen atom or a 6,6-dialkoxyhexyl group, and R³ and R⁴ are as defined above,
subsequently subjecting the nucleophilic reagent, 6,6-dialkoxyhexyl compound (7), to a nucleophilic addition reaction with 1,2-butylene oxide of the following formula (10): 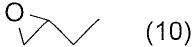 to form a 10,10-dialkoxy-3-decanol compound of the following general formula (9B): 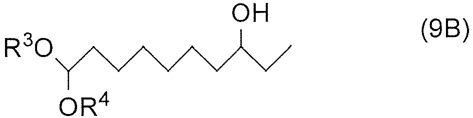 wherein R³ and R⁴ are as defined above,
subjecting the 10,10-dialkoxy-3-decanol compound (9B) to acylation to form a 10,10-dialkoxy-3-acyloxydecane compound of the following general formula (5B), which is the general formula (5) above, wherein *n* is 1: 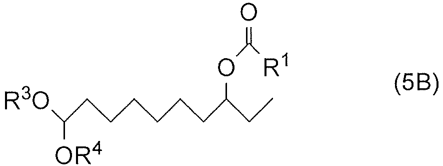 wherein R¹, R³, and R⁴ are as defined above,
subjecting the 10,10-dialkoxy-3-acyloxydecane compound (5B) to a hydrolysis reaction to form an 8-(acyloxy)alkanal compound (1), wherein *n* = 1, which is the general formula (1) above, wherein n is 1,
and the aforesaid preparation process according to the second aspect, comprising preparing the 2,9-diacetoxyundecane (4) from the 8-(acyloxy)alkanal compound (1), wherein n = 1.

According to the present invention, an 8-(acyloxy)alkanal compound, a synthetic intermediate that allows efficient synthesis of 2,9-diacetoxyundecane, may be prepared. Furthermore, 2,9-diacetoxyundecane may be prepared efficiently with fewer steps and low environmental impact by using the 8-(acyloxy)alkanal compound as a key intermediate. The preparation process is economic as well.

### DETAILED DESCRIPTION OF THE INVENTION

### I. 8-(Acyloxy)alkanal compound of the general formula (1) below

First, the 8-(acyloxy)alkanal compound (1) will be explained in detail below.

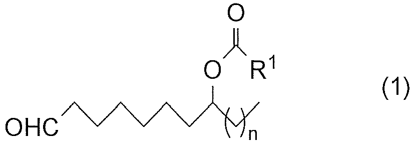

In the general formula (1) above, n represents 0 or 1.

In the present specification, the notation of "8-(acyloxy)alkanal compound (1: *n* = 0)" or "8-(acyloxy)alkanal compound (1A)" is used when *n* is 0, and the notation of "8-(acyloxy)alkanal compound (1: *n* = 1)" or "8-(acyloxy)alkanal compound (1B)" is used when *n* is 1.

In the general formula (1) above, R¹ represents a linear or branched alkyl group having 1 to 10, preferably 1 to 6, and more preferably 1, 2, 3, or 4 carbon atoms, a phenyl group, or a phenyl group in which one or more hydrogen atoms are substituted with a halogen atom.

Examples of the linear or branched alkyl group R¹ having 1 to 10 carbon atoms include a methyl group, an ethyl group, an n-propyl group, an n-butyl group, an isopropyl group, a see-butyl group, and a *tert*-butyl group.

Examples of the halogen atom of the phenyl group having one or more hydrogen atoms substituted with a halogen atom include a fluorine atom, a chlorine atom, a bromine atom, and an iodine atom. Any hydrogen atom of the phenyl group may be substituted with a halogen atom. The number of halogen atoms in the phenyl group is 1, 2, 3, 4, or 5. When multiple hydrogen atoms of the phenyl group are substituted with halogen atoms, the halogen atoms are independent of each other.

The 8-(acyloxy)alkanal compound (1A) and the 8-(acyloxy)alkanal compound (1B) will be explained below as specific examples of the 8-(acyloxy)alkanal compound (1).

Examples of the 8-(acyloxy)alkanal compound (1A) include the following compounds:
8-(acyloxy)nonanal compounds such as 8-(acetyloxy)nonanal, 8-(propionyloxy)nonanal, 8-(butyryloxy)nonanal, 8-(isobutyryloxy)nonanal, 8-(2-methylbutyryloxy)nonanal, 8-(pivaloyloxy)nonanal, and 8-(benzoyloxy)nonanal.

Examples of the 8-(acyloxy)alkanal compound (1B) include the following compounds:
8-(acyloxy)decanal compounds such as 8-(acetyloxy)decanal, 8-(propionyloxy)decanal, 8-(butyryloxy)decanal, 8-(isobutyryloxy)decanal, 8-(2-methylbutyryloxy)decanal, 8-(pivaloyloxy)decanal, and 8-(benzoyloxy)decanal.

### II. Process for preparing the aforesaid 8-(acyloxy)alkanal compound (1)

The 8-(acyloxy)alkanal compound (1), a target compound of the present invention, is prepared from a 6-halo-1, 1-dialkoxyhexane compound of the following general formula (6) according to, for example, a preparation process of the chemical reaction formula below.

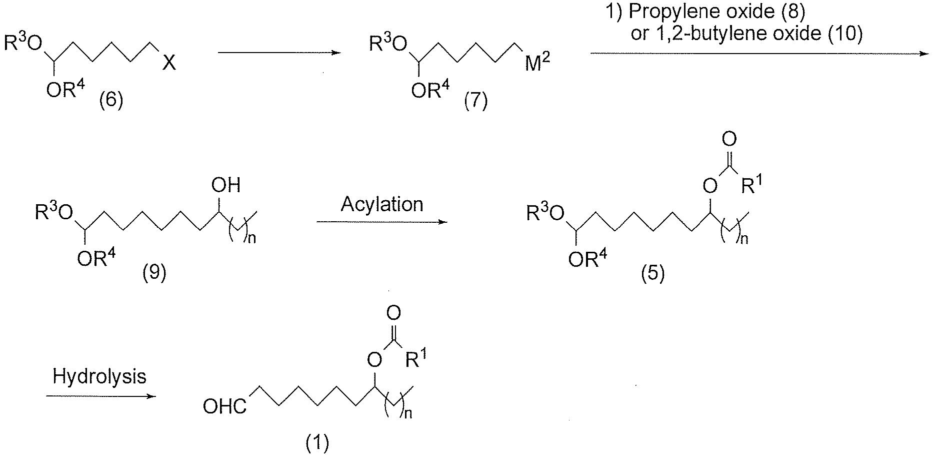

Specifically, the 8-(acyloxy)alkanal compound (1) may be prepared by (i) converting 6-halo-1,1-dialkoxyhexane compound (6) into a nucleophilic reagent, 6,6-dialkoxyhexyl compound (7), and then (ii) subjecting the nucleophilic reagent, 6,6-dialkoxyhexyl compound (7), to a nucleophilic addition reaction with propylene oxide (8) or 1,2-butylene oxide (10) to form a dialkoxyalkanol compound (9), subsequently (iii) subjecting the dialkoxyalkanol compound (9) to acylation to form a dialkoxyacyloxyalkane compound (5), and subsequently (iv) hydrolyzing the dialkoxyacyloxyalkane compound (5).
(i) Aforesaid nucleophilic reagent, 6,6-dialkoxyhexyl compound (7), and process for preparing aforesaid nucleophilic reagent, 6,6-dialkoxyhexyl compound (7)
   (a) The nucleophilic reagent, 6,6-dialkoxyhexyl compound (7), will be explained in detail below.

The nucleophilic reagent, 6,6-dialkoxyhexyl compound (7), is represented by the following general formula (7):

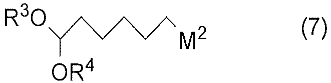

In the general formula (7) above, M² represents Li, MgZ², CuZ², or CuLiZ², and Z² represents a halogen atom or a 6,6-dialkoxyhexyl group. Examples of the halogen atom Z² include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

In the general formula (7) above, R³ and R⁴ represent, independently of each other, a monovalent hydrocarbon group having 1 to 15 carbon atoms, preferably 1 to 10 carbon atoms, more preferably 1 to 5 carbon atoms, and even more preferably 1 to 4 carbon atoms; or R³ and R⁴ form together a divalent hydrocarbon group, R³-R⁴, having 2 to 10 carbon atoms, and preferably 2 to 5 carbon atoms.

Examples of the monovalent hydrocarbon group having 1 to 15 carbon atoms include linear saturated hydrocarbon groups such as a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, and an n-dodecyl group; branched saturated hydrocarbon groups such as an isopropyl group, a 2-methylpropyl group, and a 2-methylbutyl group; linear unsaturated hydrocarbon groups such as a 2-propenyl group; branched unsaturated hydrocarbon groups such as a 2-methyl-2-propenyl group; cyclic saturated hydrocarbon groups such as a cyclopropyl group; and isomers thereof. A part of the hydrogen atoms in the hydrocarbon groups may be substituted with a methyl group or an ethyl group.

The monovalent hydrocarbon group is preferably a methyl group, an ethyl group, an n-propyl group, and an n-butyl group. By using said methyl group, ethyl group, n-propyl group, and n-butyl group, a preferred handling may be ensured.

When R³ and R⁴ form together a divalent hydrocarbon group, R³-R⁴, having 2 to 10 carbon atoms, examples of the divalent hydrocarbon group include linear saturated hydrocarbon groups such as an ethylene group, a 1,3-propylene group, and a 1,4-butylene group; branched saturated hydrocarbon groups such as a 1,2-propylene group, a 2,2-dimethyl-1,3-propylene group, a 1,2-butylene group, a 1,3-butylene group, a 2,3-butylene group, and a 2,3-dimethyl-2,3-butylene group; linear unsaturated hydrocarbon groups such as a 1-vinylethylene group; branched unsaturated hydrocarbon groups such as a 2-methylene-1,3-propylene group; cyclic hydrocarbon groups such as a 1,2-cyclopropylene group and a 1,2-cyclobutylene group; and isomers thereof. A part of the hydrogen atoms in the hydrocarbon groups may be substituted with a methyl group or an ethyl group.

In view of a preferred reactivity of deprotection, ease of purification, and/or availability, the divalent hydrocarbon group is preferably a lower hydrocarbon group, preferably having 2 to 4 carbon atoms with high reactivity and deprotection by-products that are easily removable by washing or concentration.

Therefore, the divalent hydrocarbon group is particularly preferably an ethylene group, a 1,2-propylene group, a 1,3-propylene group, a 1,2-butylene group, a 1,3-butylene group, and a 2,3-dimethyl-2,3-butylene group. By using said ethylene group, 1,2-propylene group, 1,3-propylene group, 1,2-butylene group, 1,3-butylene group, and 2,3-dimethyl-2,3-butylene group, a particularly preferred reactivity of deprotection, ease of purification, and/or availability may be ensured.

When M² is MgZ² in the general formula (7) above, the nucleophilic reagent, 6,6-dialkoxyhexyl compound (7: M² = MgZ²), is a Grignard reagent.

Specific examples of the nucleophilic reagent, 6,6-dialkoxyhexyl compound (7), include the following compounds:
6,6-dialkoxyhexyllithium compounds (when M² = Li) such as 6,6-dimethoxyhexyllithium, 6,6-diethoxyhexyllithium, 6,6-dipropyloxyhexyllithium, 6,6-dibutyloxyhexyllithium, 6,6-dipentyloxyhexyllithium, 6,6-dihexyloxyhexyllithium, 6,6-diheptyloxyhexyllithium, 6,6-dioctyloxyhexyllithium, 6,6-dinonyloxyhexyllithium, 6,6-didecyloxyhexyllithium, [5-(1,3-dioxolan-2-yl)pentyl]lithium, and [5-(1,3-dioxan-2-yl)pentyl]lithium;
6,6-dialkoxyhexylmagnesium halide compounds (when M² = MgZ²) such as 6,6-dimethoxyhexylmagnesium chloride, 6,6-diethoxyhexylmagnesium chloride, 6,6-dipropyloxyhexylmagnesium chloride, 6,6-dibutyloxyhexylmagnesium chloride, 6,6-dipentyloxyhexylmagnesium chloride, 6,6-dihexyloxyhexylmagnesium chloride, 6,6-diheptyloxyhexylmagnesium chloride, 6,6-dioctyloxyhexylmagnesium chloride, 6,6-dinonyloxyhexylmagnesium chloride, 6,6-didecyloxyhexylmagnesium chloride, [5-(1,3-dioxolan-2-yl)pentyl]magnesium chloride, [5-(1,3-dioxan-2-yl)pentyl]magnesium chloride, 6,6-dimethoxyhexylmagnesium bromide, 6,6-diethoxyhexylmagnesium bromide, 6,6-dipropyloxyhexylmagnesium bromide, 6,6-dibutyloxyhexylmagnesium bromide, 6,6-dipentyloxyhexylmagnesium bromide, 6,6-dihexyloxyhexylmagnesium bromide, 6,6-diheptyloxyhexylmagnesium bromide, 6,6-dioctyloxyhexylmagnesium bromide, 6,6-dinonyloxyhexylmagnesium bromide, 6,6-didecyloxyhexylmagnesium bromide, [5-(1,3-dioxolan-2-yl)pentyl)magnesium bromide, [5-(1,3-dioxan-2-yl)pentyl]magnesium bromide, 6,6-dimethoxyhexylmagnesium iodide, 6,6-diethoxyhexylmagnesium iodide, 6,6-dipropyloxyhexylmagnesium iodide, 6,6-dibutyloxyhexylmagnesium iodide, 6,6-dipentyloxyhexylmagnesium iodide, 6,6-dihexyloxyhexylmagnesium iodide, 6,6-diheptyloxyhexylmagnesium iodide, 6,6-dioctyloxyhexylmagnesium iodide, 6,6-dinonyloxyhexylmagnesium iodide, 6,6-didecyloxyhexylmagnesium iodide, [5-(1,3-dioxolan-2-yl)pentyl)magnesium iodide, and [5-(1,3-dioxan-2-yl)pentyl]magnesium iodide (i.e., Grignard reagents);
bis[6,6-dialkoxyhexyl]cuprate compounds (when M² = CuZ²) such as bis[6,6-dimethoxyhexyl]cuprate, bis[6,6-diethoxyhexyl]cuprate, bis[6,6-dipropyloxyhexyl]cuprate, bis[6,6-dibutyloxyhexyl]cuprate, bis[6,6-dipentyloxyhexyl]cuprate, bis[6,6-dihexyloxyhexyl]cuprate, bis[6,6-diheptyloxyhexyl]cuprate, bis[6,6-dioctyloxyhexyl]cuprate, bis[6,6-dinonyloxyhexyl]cuprate, and bis[6,6-didecyloxyhexyl]cuprate; and

Gilman reagents (when M² = CuLiZ²) such as lithium bis[6,6-dialkoxyhexyl]cuprate compounds such as lithium bis[6,6-dimethoxyhexyl]cuprate, lithium bis[6,6-diethoxyhexyl]cuprate, lithium bis[6,6-dipropyloxyhexyl]cuprate, lithium bis[6,6-dibutyloxyhexyl]cuprate, lithium bis[6,6-dipentyloxyhexyl]cuprate, lithium bis[6,6-dihexyloxyhexyl]cuprate, lithium bis[6,6-diheptyloxyhexyl]cuprate, lithium bis[6,6-dioctyloxyhexyl]cuprate, lithium bis[6,6-dinonyloxyhexyl]cuprate, and lithium bis[6,6-didecyloxyhexyl]cuprate (i.e., Gilman reagents).

Grignard reagents such as 6,6-dimethoxyhexylmagnesium halide compounds are preferred. By using said Grignard reagents such as 6,6-dimethoxyhexylmagnesium halide compounds, a preferred reactivity may be ensured.

(b) A process for preparing the aforesaid nucleophilic reagent, 6,6-dialkoxyhexyl compound (7), will be explained in detail below.

A nucleophilic reagent, 6,6-dialkoxyhexyl compound (7), may be prepared from the aforesaid 6-halo-1,1-dialkoxyhexane compound (6). The 6-halo-1,1-dialkoxyhexane compound (6) may be prepared according to, for example, the preparation process described in Japanese Patent Application Laid-Open No. 2019-189535.

A process for preparing a nucleophilic reagent, 6,6-dialkoxyhexyl compound (7), when M² is MgZ², that is, a 6,6-dialkoxyhexylmagnesium halide reagent (7: M² = MgZ²) (i.e., a Grignard reagent), will be described in detail as an example.

A 6,6-dialkoxyhexylmagnesium halide reagent (7: when M² = MgZ²) may be prepared, for example, by reacting the aforesaid 6-halo-1,1-dialkoxyhexane compound (6) with magnesium in a solvent, as shown in the following chemical reaction formula:

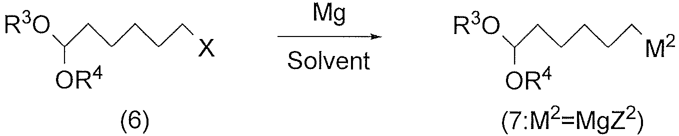

The amount of magnesium used, per mol of the 6-halo-1,1-dialkoxyhexane compound (6), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reaction rate of forming the aforesaid Grignard reagent may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reaction rate of forming the aforesaid Grignard reagent may be ensured.

The solvent may be used alone or in combination thereof, if necessary. The solvent may be a commercially available one.

The amount of the solvent used, per mol of the 6-halo-1,1-dialkoxyhexane compound (6), is preferably 30 to 5,000 g, and more preferably 50 to 3,000 g. By using said preferred amount and said more preferred amount, a preferred reactivity and a more preferred reactivity may be ensured.

The reaction temperature varies, depending on the solvent used, and is preferably 30 to 120°C. By using said preferred reaction temperature, a preferred reactivity may be ensured.

The reaction time varies, depending on the solvent used and/or production scale, and is preferably 0.5 to 100 hours. By using said preferred reaction time, a preferred reactivity may be ensured.

### (ii) Aforesaid dialkoxyalkanol compound (9) and process for preparing aforesaid dialkoxyalkanol compound (9)

### (a) The dialkoxyalkanol compound (9) will be explained in detail below.

The dialkoxyalkanol compound (9) is represented by the following general formula (9):

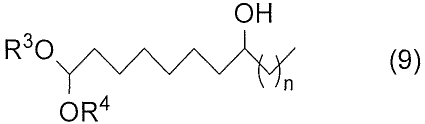

In the general formula (9) above, R³ and R⁴ are as defined for the general formula (7), and n is as defined for the general formula (1) above.

In the present specification, the notation of dialkoxyalkanol compound (9: n = 0), dialkoxyalkanol compound (9A), or 9,9-dialkoxy-2-nonanol compound (9A) is used when n is 0, and the notation of dialkoxyalkanol compound (9: *n* = 1), dialkoxyalkanol compound (9B), or 10,10-dialkoxy-3-decanol compound (9B) is used when n is 1.

A dialkoxyalkanol compound (9A) and a dialkoxyalkanol compound (9B) will be described below as specific examples of a dialkoxyalkanol compound (9). Examples of a dialkoxyalkanol compound (9A) include the following compounds:
9,9-dialkoxy-2-nonanol compounds such as 9,9-dimethoxy-2-nonanol, 9,9-diethoxy-2-nonanol, 9,9-dipropyloxy-2-nonanol, 9,9-dibutyloxy-2-nonanol, 9,9-dipentyloxy-2-nonanol, 9,9-dihexyloxy-2-nonanol, 9,9-diheptyloxy-2-nonanol, 9,9-dioctyloxy-2-nonanol, 9,9-dinonyloxy-2-nonanol, 9,9-didecyloxy-2-nonanol, α-methyl-1,3-dioxolane-2-heptanol, and α-methyl-1,3-dioxane-2-heptanol.

Examples of a dialkoxyalkanol compound (9B) include the following compounds:
10,10-dialkoxy-3-decanol compounds such as 10,10-dimethoxy-3-decanol, 10,10-diethoxy-3-decanol, 10,10-dipropyloxy-3-decanol, 10,10-dibutyloxy-3-decanol, 10,10-dipentyloxy-3-decanol, 10,10-dihexyloxy-3-decanol, 10,10-diheptyloxy-3-decanol, 10,10-dioctyloxy-3-decanol, 10,10-dinonyloxy-3-decanol, 10,10-didecyloxy-3-decanol, α**-**ethyl-1,3-dioxolane-2-heptanol, and α-ethyl-1,3-dioxane-2-heptanol.

(b) A process for preparing the aforesaid dialkoxyalkanol compound (9) will be explained in detail below.

The dialkoxyalkanol compound (9) may be prepared from the aforesaid nucleophilic reagent, 6,6-dialkoxyhexyl compound (7), as shown in the following chemical reaction formula:

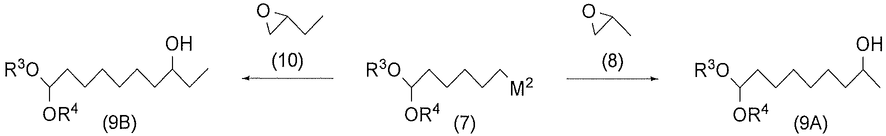

The preparation process includes at least a step of subjecting the nucleophilic reagent, 6,6-dialkoxyhexyl compound (7), to a nucleophilic addition reaction with propylene oxide of the general formula (8) above or 1,2-butylene oxide of the general formula (10) above to respectively form the dialkoxyalkanol compound (9A) or (9B).

In the aforesaid nucleophilic addition reaction, the nucleophilic reagent, 6,6-dialkoxyhexyl compound (7), may be used alone or in combination thereof, if necessary.

Propylene oxide (8) and 1,2-butylene oxide (10) each may be a commercially available one, or may be synthesized in house.

In the nucleophilic addition reaction, the amount of the nucleophilic reagent, 6,6-dialkoxyhexyl compound (7), used, per mol of propylene oxide (8) or 1,2-butylene oxide (10), is preferably 0.6 to 1.3 mol. By using said preferred amount, preferred economy may be ensured.

A solvent may be incorporated in the nucleophilic addition reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N,N-*dimethylacetamide (DMAC), N-methylpyrrolidone (NMP), dimethyl sulfoxide (DMSO), γ-butyrolactone (GBL), acetonitrile, *N*,*N*'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of the nucleophilic reagent, 6,6-dialkoxyhexyl compound (7), is preferably 30 to 5,000 g, and more preferably 50 to 3,000 g. By using said preferred amount and said more preferred amount, a preferred reactivity and a more preferred reactivity may be ensured.

The nucleophilic addition reaction may be carried out in the presence of a catalyst, if necessary. Examples of the catalyst include cuprous halides such as cuprous chloride, cuprous bromide, and cuprous iodide; and cupric halides such as cupric chloride, cupric bromide, and cupric iodide. Cuprous halides are preferred. By using said cuprous halides, a preferred reactivity may be ensured. Cuprous chloride is more preferred. By using said cuprous chloride, a more preferred reactivity may be ensured.

The catalyst may be used alone or in combination thereof, if necessary. The catalyst may be a commercially available one.

The amount of the catalyst used, per mol of the nucleophilic reagent, 6,6-dialkoxyhexyl compound (7), is preferably 0.0001 to 0.300 mol, and more preferably 0.0003 to 0.100 mol. By using said preferred amount and said more preferred amount, a preferred reaction rate and/or post-treatment and a more preferred reaction rate and/or post-treatment may be ensured.

When the nucleophilic addition reaction is carried out in the presence of the catalyst, a co-catalyst may be added, if necessary. Examples of the co-catalyst include phosphorus compounds such as trialkyl phosphite compounds having 3 to 9 carbon atoms such as triethyl phosphite; and triarylphosphine compounds having 18 to 21 carbon atoms such as triphenylphosphine. Trialkyl phosphite compounds having 3 to 9 carbon atoms, which are liquids at a room temperature, are preferred. By using said trialkyl phosphite compounds having 3 to 9 carbon atoms, a preferred handling may be ensured.

The co-catalyst may be used alone or in combination thereof, if necessary. The co-catalyst may be a commercially available one.

When the co-catalyst is used, the amount of the co-catalyst used, per mol of the nucleophilic reagent, 6,6-dialkoxyhexyl compound (7), is preferably more than 0 up to 0.500 mol, and more preferably more than 0 up to 0.200 mol. By using said preferred amount and said more preferred amount, a preferred reactivity and a more preferred reactivity may be ensured.

When the nucleophilic addition reaction is carried out in the presence of the catalyst, a lithium halide may be added, if necessary.

Examples of the lithium halide include lithium chloride, lithium bromide, and lithium iodide. Lithium chloride is preferred. By using said lithium chloride, a preferred reactivity may be ensured.

The lithium halide may be used alone or in combination thereof, if necessary. The lithium halide may be a commercially available one.

When the lithium halide is used, the amount of the lithium halide used, per mol of the nucleophilic reagent, 6,6-dialkoxyhexyl compound (7), is preferably more than 0 up to 0.250 mol. By using said preferred amount, a preferred reactivity may be ensured.

The reaction temperature of the nucleophilic addition reaction varies, depending on the nucleophilic reagent, 6,6-dialkoxyhexyl compound (7), used, and is preferably -78 to 70°C, more preferably -20 to 50°C, and even more preferably 5 to 35°C. By using said preferred reaction temperature, said more preferred reaction temperature, and said even more preferred reaction temperature, a preferred reactivity, a more preferred reactivity, and an even more preferred reactivity may be ensured.

The reaction time of the nucleophilic addition reaction varies, depending on the solvent used and/or production scale, and is preferably 0.5 to 100 hours. By using said preferred reaction time, a preferred reactivity may be ensured.

### (iii) Aforesaid dialkoxyacyloxyalkane compound (5) and process for preparing aforesaid dialkoxyacyloxyalkane compound (5)

(a) The dialkoxyacyloxyalkane compound (5) is represented by the following general formula (5):

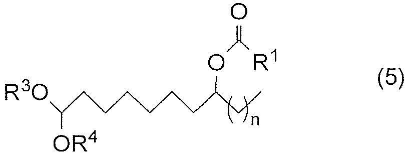

In the general formula (5) above, R³ and R⁴ are as defined for the general formula (7), and n and R¹ are as defined for the general formula (1).

In the present specification, the notation of 9,9-dialkoxy-2-acyloxynonane compound (5: *n* = 0) or 9,9-dialkoxy-2-acyloxynonane compound (5A) is used when *n* is 0, and the notation of 10,10-dialkoxy-3-acyloxydecane compound (5: n = 1) or 10,10-dialkoxy-3-acyloxydecane compound (5B) is used when *n* is 1.

A 9,9-dialkoxy-2-acyloxynonane compound (5A) and a 10,10-dialkoxy-3-acyloxydecane compound (5B) will be described below as specific examples of a dialkoxyacyloxyalkane compound (5).

Examples of a 9,9-dialkoxy-2-acyloxynonane compound (5A) include the following compounds:
9,9-dialkoxy-2-acetyloxynonane compounds such as 9,9-dimethoxy-2-acetyloxynonane, 9,9-diethoxy-2-acetyloxynonane, 9,9-dipropyloxy-2-acetyloxynonane, 9,9-dibutyloxy-2-acetyloxynonane, 9,9-dipentyloxy-2-acetyloxynonane, 9,9-dihexyloxy-2-acetyloxynonane, 9,9-diheptyloxy-2-acetyloxynonane, 9,9-dioctyloxy-2-acetyloxynonane, 9,9-dinonyloxy-2-acetyloxynonane, 9,9-didecyloxy-2-acetyloxynonane, α-methyl-1,3-dioxolane-2-heptyl acetate, and α-methyl-1,3-dioxane-2-heptyl acetate;
9,9-dialkoxy-2-pivaloyloxynonane compounds such as 9,9-dimethoxy-2-pivaloyloxynonane, 9,9-diethoxy-2-pivaloyloxynonane, 9,9-dipropyloxy-2-pivaloyloxynonane, 9,9-dibutyloxy-2-pivaloyloxynonane, 9,9-dipentyloxy-2-pivaloyloxynonane, 9,9-dihexyloxy-2-pivaloyloxynonane, 9,9-diheptyloxy-2-pivaloyloxynonane, 9,9-dioctyloxy-2-pivaloyloxynonane, 9,9-dinonyloxy-2-pivaloyloxynonane, and 9,9-didecyloxy-2-pivaloyloxynonane; and
9,9-dialkoxy-2-benzyloxynonane compounds such as 9,9-dimethoxy-2-benzyloxynonane, 9,9-diethoxy-2-benzyloxynonane, 9,9-dipropyloxy-2-benzyloxynonane, 9,9-dibutyloxy-2-benzyloxynonane, 9,9-dipentyloxy-2-benzyloxynonane, 9,9-dihexyloxy-2-benzyloxynonane, 9,9-diheptyloxy-2-benzyloxynonane, 9,9-dioctyloxy-2-benzyloxynonane, 9,9-dinonyloxy-2-benzyloxynonane, and 9,9-didecyloxy-2-benzyloxynonane.

Examples of a 10,10-dialkoxy-3-acyloxydecane compound (5B) include the following compounds:
10,10-dialkoxy-3-acetyloxydecane compounds such as 10,10-dimethoxy-3-acetyloxydecane, 10,10-diethoxy-3 -acetyloxydecane, 10,10-dipropyloxy-3-acetyloxydecane, 10,10-dibutyloxy-3-acetyloxy decane, 10,10-dipentyloxy-3-acetyloxydecane, 10,10-dihexyloxy-3-acetyloxydecane, 10,10-diheptyloxy-3-acetyloxydecane, 10,10-dioctyloxy-3-acetyloxy decane, 10,10-dinonyloxy-3-acetyloxydecane, 10,10-didecyloxy-3-acetyloxydecane, α-ethyl-1,3-dioxolane-2-heptyl acetate, and α-ethyl-1,3-dioxane-2-heptyl acetate;
10,10-dialkoxy-3-pivaloyloxydecane compounds such as 10,10-dimethoxy-3-pivaloyloxydecane, 10,10-diethoxy-3-pivaloyloxydecane, 10,10-dipropyloxy-3-pivaloyloxydecane, 10,10-dibutyloxy-3-pivaloyloxydecane, 10,10-dipentyloxy-3-pivaloyloxydecane, 10,10-dihexyloxy-3-pivaloyloxydecane, 10,10-diheptyloxy-3-pivaloyloxydecane, 10,10-dioctyloxy-3-pivaloyloxydecane, 10,10-dinonyloxy-3-pivaloyloxydecane, and 10,10-didecyloxy-3-pivaloyloxydecane; and
10,10-dialkoxy-3-benzyloxydecane compounds such as 10,10-dimethoxy-3-benzyloxydecane, 10,10-diethoxy-3-benzyloxydecane, 10,10-dipropyloxy-3-benzyloxydecane, 10,10-dibutyloxy-3-benzyloxydecane, 10,10-dipentyloxy-3-benzyloxydecane, 10,10-dihexyloxy-3-benzyloxy decane, 10,10-diheptyloxy-3-benzyloxydecane, 10,10-dioctyloxy-3-benzyloxy decane, 10,10-dinonyloxy-3-benzyloxydecane, and 10,10-didecyloxy-3-benzyloxydecane.

(b) A process for preparing the aforesaid dialkoxyacyloxyalkane compound (5) will be explained in detail below.

The dialkoxyacyloxyalkane compound (5) may be prepared, for example, from the aforesaid dialkoxyalkanol compound (9), as shown in the following chemical reaction formula:

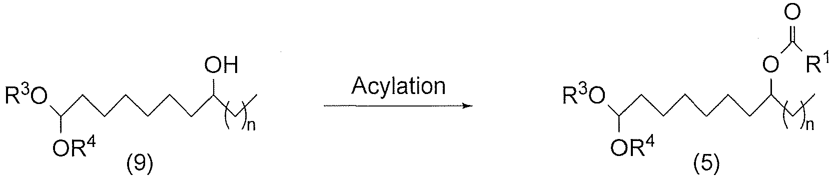

In the general formula (5) above, R³ and R⁴ are as defined for the general formula (7), and n and R¹ are as defined for the general formula (1).

The preparation process includes a step of subjecting the dialkoxyalkanol compound (9) to acylation.

The acylation may be carried out, for example, by acylating a hydroxy group of the dialkoxyalkanol compound (9) with an acylating agent.

Examples of the acylating agent include the following compounds:
carboxylic acids such as acetic acid, propanoic acid, butanoic acid, pivalic acid, and benzoic acid;
acid anhydrides such as acetic anhydride, pivalic anhydride, and benzoic anhydride;
acyl halide compounds such as acetyl chloride, acetyl bromide, acetyl iodide, propanoyl chloride, propanoyl bromide, propanoyl iodide, butyryl chloride, butyryl bromide, butyryl iodide, pivaloyl chloride, pivaloyl bromide, pivaloyl iodide, benzoyl chloride, benzoyl bromide, and benzoyl iodide;
alkanoic acid ester compounds such as methyl acetate, ethyl acetate, methyl propanoate, ethyl propanoate, methyl butanoate, ethyl butanoate, methyl pivalate, and ethyl pivalate; and
benzoic acid ester compounds such as methyl benzoate and ethyl benzoate.

The acylating agent is preferably acid anhydrides and acyl halide compounds. By using said acid anhydrides and acyl halide compounds, a preferred reactivity may be ensured. Acid anhydrides are more preferred. By using said acid anhydrides, a more preferred reactivity may be ensured.

The amount of the acylating agent used, per mol of the dialkoxyalkanol compound (9), is preferably 1.0 to 10.0 mol, and more preferably 1.0 to 5.0 mol. By using said preferred amount and said more preferred amount, a preferred reactivity and/or economy and a more preferred reactivity and/or economy may be ensured.

An acid or a base may be incorporated in the acylation, if necessary.

Examples of the acid include mineral acids such as hydrochloric acid, sulfuric acid, and nitric acid; aromatic sulfonic acids such as benzenesulfonic acid and p-toluenesulfonic acid; and Lewis acids such as aluminum trichloride, aluminum ethoxide, aluminum isopropoxide, aluminum oxide, boron trifluoride, boron trichloride, boron tribromide, magnesium chloride, magnesium bromide, magnesium iodide, zinc chloride, zinc bromide, zinc iodide, tin tetrachloride, tin tetrabromide, dibutyltin dichloride, dibutyltin dimethoxide, dibutyltin oxide, titanium tetrachloride, titanium tetrabromide, titanium(IV) methoxide, titanium(IV) ethoxide, titanium(IV) isopropoxide, and titanium(IV) oxide.

The acid may be used alone or in combination thereof, if necessary.

When the acid is used, the amount of the acid used, per mol of the dialkoxyalkanol compound (9), is preferably more than 0 up to 3.00 mol, and more preferably more than 0 up to 1.50 mol. By using said preferred amount and said more preferred amount, a preferred reactivity and/or economy and a more preferred reactivity and/or economy may be ensured.

Examples of the base include the following compounds:
trialkylamine compounds such as trimethylamine, triethylamine, and *N,N-*diisopropylethylamine;
cyclic amine compounds such as piperidine, pyrrolidine, and 1,8-diazabicyclo[5.4.0]-7-undecene (DBU);
aromatic amine compounds such as pyridine, lutidine, *N,N*-dimethylaniline, N,N-diethylaniline, *N,N*-dibutylaniline, and 4-dimethylaminopyridine;
metal alkoxides such as sodium methoxide, sodium ethoxide, sodium t-butoxide, sodium t-amyloxide, lithium methoxide, lithium ethoxide, lithium t-butoxide, lithium t-amyloxide, potassium methoxide, potassium ethoxide, potassium t-butoxide, and potassium t-amyloxide;
alkyllithiums such as methyllithium, ethyllithium, propyllithium, n-butyllithium, sec-butyllithium, tert-butyllithium, pentyllithium, hexyllithium, heptyllithium, octyllithium, nonyllithium, and decyllithium;
Grignard reagents such as methylmagnesium chloride, methylmagnesium bromide, methylmagnesium iodide, ethylmagnesium chloride, ethylmagnesium bromide, ethylmagnesium iodide, propylmagnesium chloride, propylmagnesium bromide, propylmagnesium iodide, butylmagnesium chloride, butylmagnesium bromide, butylmagnesium iodide, pentylmagnesium chloride, pentylmagnesium bromide, pentylmagnesium iodide, hexylmagnesium chloride, hexylmagnesium bromide, hexylmagnesium iodide, heptylmagnesium chloride, heptylmagnesium bromide, heptylmagnesium iodide, octylmagnesium chloride, octylmagnesium bromide, octylmagnesium iodide, nonylmagnesium chloride, nonylmagnesium bromide, nonylmagnesium iodide, decylmagnesium chloride, decylmagnesium bromide, and decylmagnesium iodide; and
metal acetylides such as lithium acetylide, sodium acetylide, potassium acetylide, calcium acetylide, and silver acetylide.

The base may be used alone or in combination thereof, if necessary.

When the base is used, the amount of the base used, per mol of the dialkoxyalkanol compound (9), is preferably more than 0 up to 10.0 mol, and more preferably more than 0 up to 5.0 mol. By using said preferred amount and said more preferred amount, a preferred reactivity and/or economy and a more preferred reactivity and/or economy may be ensured.

A solvent may be incorporated in the acylation, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N-*dimethylformamide (DMF), *N,N-*dimethylacetamide (DMAC), *N*-methylpyrrolidone (NMP), dimethyl sulfoxide (DMSO), γ-butyrolactone (GBL), acetonitrile, dichloromethane, chloroform, and hexamethylphosphoric triamide (HMPA). Ether solvents such as tetrahydrofuran, 2-methyltetrahydrofuran, and 4-methyltetrahydropyran; and hydrocarbon solvents such as toluene and xylene are preferred. By using said ether solvents such as tetrahydrofuran, 2-methyltetrahydrofuran, and 4-methyltetrahydropyran; and hydrocarbon solvents such as toluene and xylene, a preferred reactivity may be ensured.

The solvent may be used alone or in combination thereof, if necessary. The solvent may be a commercially available one.

A solvent may be incorporated in the acylation, if necessary, or alternatively this acylation may also be carried out without a solvent.

The amount of the solvent used in the acylation, per mol of the aforesaid dialkoxyalkanol compound (9), is preferably more than 0 up to 5,000 g, and more preferably more than 0 up to 2,000 g. By using said preferred amount and said more preferred amount, a preferred reactivity and a more preferred reactivity may be ensured.

The reaction temperature of the acylation varies, depending on the acylating agent and/or solvent used, and is preferably -40 to 120°C, more preferably -20 to 100°C, and even more preferably 0 to 80°C. By using said preferred reaction temperature, said more preferred reaction temperature, and said even more preferred reaction temperature, a preferred reactivity, a more preferred reactivity, and an even more preferred reactivity may be ensured.

The reaction time of the acylation is preferably 0.5 to 100 hours. By using said preferred reaction time, a preferred reactivity may be ensured.

In the preparation of the dialkoxyalkanol compound (9) and the preparation of the dialkoxyacyloxyalkane compound (5), the nucleophilic substitution reaction and the acylation may be carried out separately, or the acylation may be carried out continuously from the nucleophilic substitution reaction (see Examples 3 and 4 below). A continuous reaction is more preferred. By carrying out said continuous reaction, a more preferred solvent conservation and/or reaction time reduction may be ensured.

### (iv) Aforesaid 8-(acyloxy)alkanal compound (1) and process for preparing aforesaid 8-(acyloxy)alkanal compound (1)

(a) The 8-(acyloxy)alkanal compound (1) is as mentioned above.
(b) A process for preparing the 8-(acyloxy)alkanal compound (1) will be explained in detail below.

The 8-(acyloxy)alkanal compound (1) may be prepared from the aforesaid dialkoxyacyloxyalkane compound (5), as shown in the following chemical reaction formula:

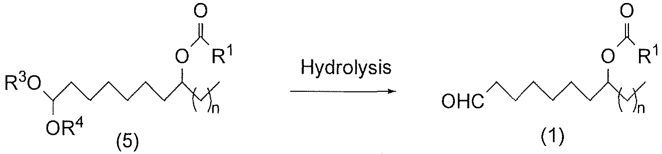

The process for preparing the 8-(acyloxy)alkanal compound (1) includes a step of subjecting a dialkoxyacyloxyalkane compound (5) to a hydrolysis reaction.

In the aforesaid hydrolysis reaction, the dialkoxyacyloxyalkane compound (5) may be used alone or in combination thereof, if necessary.

The hydrolysis reaction may be carried out, for example, with an acid and water.

Examples of the acid include inorganic acids such as hydrochloric acid and hydrobromic acid; and p-toluenesulfonic acid, benzenesulfonic acid, trifluoroacetic acid, acetic acid, formic acid, oxalic acid, iodotrimethylsilane, and titanium tetrachloride. Acetic acid, formic acid, oxalic acid, *p*-toluenesulfonic acid, and benzenesulfonic acid are preferred. By using said acetic acid, formic acid, oxalic acid, p-toluenesulfonic acid, and benzenesulfonic acid, a preferred reactivity may be ensured. *p*-toluenesulfonic acid and benzenesulfonic acid are more preferred. By using said p-toluenesulfonic acid and benzenesulfonic acid, a more preferred reaction yield may be ensured.

The acid may be used alone or in combination thereof, if necessary. The acid may be a commercially available one.

The amount of the acid used, per mol of the dialkoxyacyloxyalkane compound (5), is preferably 0.01 to 10.0 mol.

The amount of the water used, per mol of the dialkoxyacyloxyalkane compound (5), is preferably 18 to 7,000 g, and more preferably 18 to 3,000 g. By using said preferred amount and said more preferred amount, a preferred reactivity and a more preferred reactivity may be ensured.

In the hydrolysis reaction, a solvent may be further incorporated, if necessary, in addition to the acid or water.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N*,*N*-dimethylformamide (DMF), N,N-dimethylacetamide (DMAC), N-methylpyrrolidone (NMP), dimethyl sulfoxide (DMSO), γ-butyrolactone (GBL), acetonitrile, acetone, *N*,*N*'-dimethylpropylene urea (DMPU), hexamethylphosphoric triamide (HMPA), dichloromethane, and chloroform; ester solvents such as methyl acetate, ethyl acetate, n-propyl acetate, and n-butyl acetate; and alcoholic solvents such as methanol and ethanol.

The solvent may be used alone or in combination thereof, if necessary. The solvent may be a commercially available one.

The optimal solvent varies, depending on the acid to be used. When, for example, oxalic acid is used as the acid, tetrahydrofuran, 2-methyltetrahydrofuran, acetone, and γ-butyrolactone are preferred. By using said tetrahydrofuran, 2-methyltetrahydrofuran, acetone, and γ-butyrolactone, a preferred reactivity may be ensured. When *p-*toluenesulfonic acid or benzenesulfonic acid is used as the acid, hydrocarbon solvents such as toluene and xylene; and alcoholic solvents such as methanol and ethanol are preferred. By using said hydrocarbon solvents such as toluene and xylene; and alcoholic solvents such as methanol and ethanol, a preferred reactivity may be ensured.

The amount of the solvent used, per mol of the dialkoxyacyloxyalkane compound (5), is preferably more than 0 up to 7,000 g, and more preferably more than 0 up to 3,000 g. By using said preferred amount and said more preferred amount, a preferred reactivity and a more preferred reactivity may be ensured.

The reaction temperature of the hydrolysis reaction varies, depending on the acid and/or the solvent used, and is preferably -15 to 180°C, and more preferably 5 to 120°C. By using said preferred reaction temperature and said more preferred reaction temperature, a preferred reactivity and a more preferred reactivity may be ensured.

The reaction time of the hydrolysis reaction varies, depending on the acid and the solvent used and/or production scale, and is preferably 0.5 to 100 hours. By using said preferred reaction time, a preferred reactivity may be ensured.

Thus, an 8-(acyloxy)alkanal compound (1), a synthetic intermediate useful in the preparation of 2,9-diacetoxyundecane of the following general formula (4), may be prepared.

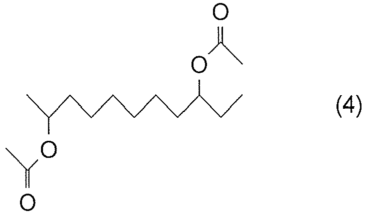

Initially, however, instead of the aforesaid 8-(acyloxy)alkanal compound (1), efforts had been focused on using a 8-hydroxyalkanal compound, the 8-(acyloxy)alkanal compound (1) without acyl group protection of the hydroxy group, as an intermediate for the preparation of the 2,9-diacetoxyundecane (4) (see Formula (11) according to Comparative Example 1 below) because it was considered pointless to protect the hydroxy group once with an acyl group and then eliminate the acyl group to free the hydroxy group. However, it became clear that the hydroxy group and the formyl group reacted to greatly reduce the yield when synthesizing the 8-hydroxyalkanal compound (11) (see Comparative Example 1 below). Therefore, although appearing to be a roundabout technique, studies made clear that in view of the yield, it is extremely important to obtain aldehyde by hydrolyzing acetal with the hydroxy group in a protected state.

### III. Process for preparing 2,9-diacetoxyundecane

2,9-Diacetoxyundecane (4), a target compound of the present invention, may be prepared, for example, from the aforesaid 8-(acyloxy)alkanal compound (1), as shown in the following chemical reaction formula:

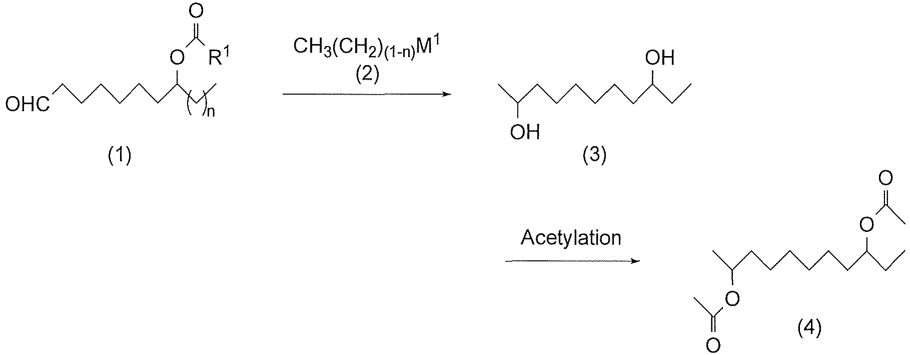

Specifically, 2,9-diacetoxyundecane (4) may be prepared by (i) subjecting the 8-(acyloxy)alkanal compound (1) to a nucleophilic addition reaction with a nucleophilic reagent, alkyl compound (2), to carry out nucleophilic addition with the formyl group and eliminate the acyl group to form 2,9-undecanediol (3), and subsequently (ii) acetylating the 2,9-undecanediol (3).

### (i) Process for preparing the aforesaid 2,9-undecanediol (3)

(a) A process for preparing the 2,9-undecanediol (3) will be explained in detail below.

The 2,9-undecanediol (3) may be prepared from the aforesaid 8-(acyloxy)alkanal compound (1), as shown in the following chemical reaction formula:

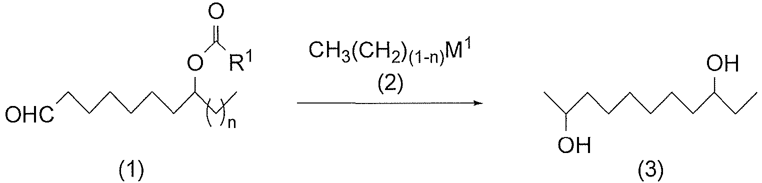

The preparation process includes a step of subjecting the 8-(acyloxy)alkanal compound (1) to a nucleophilic addition reaction with a nucleophilic reagent, alkyl compound, of the general formula (2) above.

The nucleophilic reagent, alkyl compound (2), will be explained in detail below.

In the general formula (2), M¹ represents Li, MgZ¹, CuZ¹, or CuLiZ¹, Z¹ represents a halogen atom, a methyl group, or an ethyl group, and n is as defined for the general formula (1) above.

Specific examples of the nucleophilic reagent, alkyl compound (2), include the following compounds:
alkyllithium compounds (when M¹ = Li) such as methyllithium and ethyllithium;
alkylmagnesium halide compounds (when M¹ = MgZ¹) such as methylmagnesium chloride, methylmagnesium bromide, methylmagnesium iodide, ethylmagnesium chloride, ethylmagnesium bromide, and ethylmagnesium iodide (i.e., Grignard reagents);
dialkylcuprate compounds (when M¹ = CuZ¹) such as dimethylcuprate and diethylcuprate; and
Gilman reagents (when M¹ = CuLiZ¹) such as lithium dialkylcuprate compounds such as lithium dimethylcuprate and lithium diethylcuprate (i.e., Gilman reagents).

In the aforesaid nucleophilic addition reaction, the nucleophilic reagent, alkyl compound (2), may be used alone or in combination thereof, if necessary.

In the nucleophilic addition reaction, the amount of the nucleophilic reagent, alkyl compound (2), used, per mol of the 8-(acyloxy)alkanal compound (1), is preferably 3.0 to 15.0 mol, more preferably 3.0 to 10.0 mol, and even more preferably 3.1 to 6.0 mol. By using said preferred amount, said more preferred amount, and said even more preferred amount, a preferred reactivity, a more preferred reactivity, and an even more preferred reactivity may be ensured.

A solvent may be incorporated in the nucleophilic addition reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N,N-*dimethylacetamide (DMAC), *N*-methylpyrrolidone (NMP), dimethyl sulfoxide (DMSO), γ-butyrolactone (GBL), acetonitrile, *N*,*N*'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of the aforesaid 8-(acyloxy)alkanal compound (1), is preferably 30 to 5,000 g, and more preferably 50 to 3,000 g. By using said preferred amount and said more preferred amount, a preferred reactivity and a more preferred reactivity may be ensured.

The reaction temperature of the nucleophilic addition reaction varies, depending on the 8-(acyloxy)alkanal compound (1) used, and is preferably -78 to 150°C, more preferably -20 to 100°C, and even more preferably 20 to 70°C. By using said preferred reaction temperature, said more preferred reaction temperature, and said even more preferred reaction temperature, a preferred reactivity, a more preferred reactivity, and an even more preferred reactivity may be ensured.

The reaction time of the nucleophilic addition reaction varies, depending on the solvent used and/or production scale, and is preferably 0.5 to 100 hours. By using said preferred reaction time, a preferred reactivity may be ensured.

### (ii) Process for preparing the aforesaid 2,9-diacetoxyundecane (4)

(a) A process for preparing 2,9-diacetoxyundecane (4) will be explained in detail below.

The 2,9-diacetoxyundecane (4) may be prepared from the aforesaid 2,9-undecanediol (3).

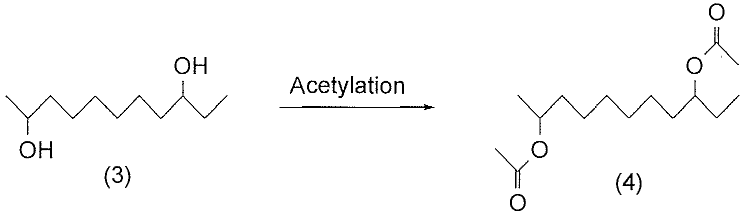

The preparation process includes a step of subjecting a 2,9-undecanediol (3) to an acetylation reaction.

The acetylation reaction may be carried out by, for example, acetylating the hydroxy group of the 2,9-undecanediol (3) with an acetylating agent.

Examples of the acetylating agent include the following compounds:
acetic acid;
acid anhydrides such as acetic anhydride;
acetyl halide compounds such as acetyl chloride, acetyl bromide, and acetyl iodide; and
acetate compounds such as methyl acetate, ethyl acetate, propyl acetate, butyl acetate, pentyl acetate, and hexyl acetate.

The acetylating agent is preferably acid anhydrides and acetyl halide compounds. By using said acid anhydrides and acetyl halide compounds, a preferred reactivity may be ensured. The acetylating agent is more preferably acid anhydrides. By using said acid anhydrides, a more preferred reactivity may be ensured.

The amount of the acylating agent used, per mol of the 2,9-undecanediol (3), is preferably 2.0 to 15.0 mol, more preferably 2.0 to 10.0 mol, and even more preferably 2.1 to 8.0 mol. By using said preferred amount, said more preferred amount, and said even more preferably amount, a preferred reactivity and/or economy, a more preferred reactivity and/or economy, and an even more preferred reactivity and/or economy may be ensured.

An acid or a base may be incorporated in the acetylation, if necessaiy.

Examples of the acid include mineral acids such as hydrochloric acid, sulfuric acid, and nitric acid; aromatic sulfonic acids such as benzenesulfonic acid and p-toluenesulfonic acid; and Lewis acids such as aluminum trichloride, aluminum ethoxide, aluminum isopropoxide, aluminum oxide, boron trifluoride, boron trichloride, boron tribromide, magnesium chloride, magnesium bromide, magnesium iodide, zinc chloride, zinc bromide, zinc iodide, tin tetrachloride, tin tetrabromide, dibutyltin dichloride, dibutyltin dimethoxide, dibutyltin oxide, titanium tetrachloride, titanium tetrabromide, titanium(IV) methoxide, titanium(IV) ethoxide, titanium(IV) isopropoxide, and titanium(IV) oxide.

The acid may be used alone or in combination thereof, if necessary.

When the acid is used, the amount of the acid used, per mol of the 2,9-undecanediol (3), is preferably more than 0 up to 3.00 mol, and more preferably more than 0 up to 1.50 mol. By using said preferred amount and said more preferred amount, a preferred reactivity and/or economy and a more preferred reactivity and/or economy may be ensured.

Examples of the base include the following compounds:
trialkylamine compounds such as trimethylamine, triethylamine, and *N,N-*diisopropylethylamine;
cyclic amine compounds such as piperidine, pyrrolidine, and 1,8-diazabicyclo[5.4.0]-7-undecene (DBU);
aromatic amine compounds such as pyridine, lutidine, *N,N*-dimethylaniline, N,N-diethylaniline, *N,N*-dibutylaniline, and 4-dimethylaminopyridine;
metal alkoxides such as sodium methoxide, sodium ethoxide, sodium t-butoxide, sodium t-amyloxide, lithium methoxide, lithium ethoxide, lithium t-butoxide, lithium t-amyloxide, potassium methoxide, potassium ethoxide, potassium t-butoxide, and potassium t-amyloxide;
alkyllithiums such as methyllithium, ethyllithium, propyllithium, n-butyllithium, sec-butyllithium, tert-butyllithium, pentyllithium, hexyllithium, heptyllithium, octyllithium, nonyllithium, and decyllithium;
Grignard reagents such as methylmagnesium chloride, methylmagnesium bromide, methylmagnesium iodide, ethylmagnesium chloride, ethylmagnesium bromide, ethylmagnesium iodide, propylmagnesium chloride, propylmagnesium bromide, propylmagnesium iodide, butylmagnesium chloride, butylmagnesium bromide, butylmagnesium iodide, pentylmagnesium chloride, pentylmagnesium bromide, pentylmagnesium iodide, hexylmagnesium chloride, hexylmagnesium bromide, hexylmagnesium iodide, heptylmagnesium chloride, heptylmagnesium bromide, heptylmagnesium iodide, octylmagnesium chloride, octylmagnesium bromide, octylmagnesium iodide, nonylmagnesium chloride, nonylmagnesium bromide, nonylmagnesium iodide, decylmagnesium chloride, decylmagnesium bromide, and decylmagnesium iodide;
metal acetylides such as lithium acetylide, sodium acetylide, potassium acetylide, calcium acetylide, and silver acetylide; and
inorganic metal salts such as potassium carbonate, sodium carbonate, and calcium carbonate.

The base may be used alone or in combination thereof, if necessary.

When the base is used, the amount of the base used, per mol of the dialkoxyalkanol compound (9), is preferably more than 0 up to 10.0 mol, and more preferably more than 0 up to 5.0 mol. By using said preferred amount and said more preferred amount, a preferred reactivity and/or economy and a more preferred reactivity and/or economy may be ensured.

A general solvent may be incorporated in the acetylation, if necessary.

Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N*-dimethylacetamide (DMAC), *N-*methylpyrrolidone (NMP), dimethyl sulfoxide (DMSO), γ-butyrolactone, acetonitrile, dichloromethane, chloroform, and hexamethylphosphoric triamide (HMPA). Ether solvents such as tetrahydrofuran, 2-methyltetrahydrofuran, and 4-methyltetrahydropyran; and hydrocarbon solvents such as toluene and xylene are preferred. By using said ether solvents such as tetrahydrofuran, 2-methyltetrahydrofuran, and 4-methyltetrahydropyran; and hydrocarbon solvents such as toluene and xylene, a preferred reactivity may be ensured.

The solvent may be used alone or in combination thereof, if necessary. The solvent may be a commercially available one.

A solvent may be incorporated in the acetylation, if necessary, or alternatively this acetylation may also be carried out without a solvent.

The amount of the solvent used in the acetylation, per mol of the aforesaid 2,9-undecanediol (3), is preferably more than 0 up to 5,000 g, and more preferably more than 0 up to 2,000 g. By using said preferred amount and said more preferred amount, a preferred reactivity and a more preferred reactivity may be ensured.

The reaction temperature of the acetylation varies, depending on the acylating agent and/or the solvent used, and is preferably -40 to 100°C, more preferably -20 to 80°C, and even more preferably 0 to 80°C. By using said preferred reaction temperature, said more preferred reaction temperature, and said even more preferred reaction temperature, a preferred reactivity, a more preferred reactivity, and an even more preferred reactivity may be ensured.

The reaction time of the acetylation is preferably 0.5 to 100 hours. By using said preferred reaction time, a preferred reactivity may be ensured.

In the preparation of the 2,9-undecanediol (3) and the preparation of the 2,9-diacetoxyundecane (4), the nucleophilic substitution reaction and the acetylation reaction may be carried out separately, or the acetylation reaction may be carried out continuously from the nucleophilic substitution reaction (see Examples 7 and 8 below). By carrying out said continuous reaction, solvent conservation and/or reaction time reduction may be ensured, and the yield of the 2,9-diacetoxyundecane (4) in two steps is increased because contact with water of 2,9-undecanediol (3), which is water-soluble, may be avoided.

Thus, according to the present invention, the 8-(acyloxy)alkanal compound (1), a synthetic intermediate, may be simply and efficiently prepared. According to the present invention, 2,9-diacetoxyundecane (4), the sex pheromone of the Swede midge (scientific name: *Contarinia nasturtii*), may be efficiently prepared with high ease of preparation and in fewer steps. Specifically, by continuously carrying out acylation and acetylation steps, 2,9-diacetoxyundecane (4) may be prepared with high yield in four steps from a 6-halo-1,1-dialkoxyhexane compound (6).

### EXAMPLES

The present invention will be described with reference to the following Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 µm × 0.25 mmφ × 30 m; carrier gas: He (1.55 mL/min), detector: FID; column temperature: 150°C, elevated in a rate of 5°C/min, and up to 230°C; column: DB-WAX, 0.25 µm × 0.25 mmφ × 30 m; carrier gas: He (1.55 mL/min), detector: FID; column temperature: 150°C, elevated in a rate of 5°C/min, and up to 230°C.

The yield was calculated according to the following equation in consideration of purities (% GC) of a starting material and a product. Yield (%) = {[(weight of a product obtained by a reaction × % GC) / molecular weight of a product] ÷ [(weight of a starting material in a reaction × % GC) / molecular weight of a starting material] × 100

THF represents tetrahydrofuran, Ph represents a phenyl group, Ts represents a toluenesulfonyl group, Ac represents an acetyl group, Bu represents an n-butyl group, ^{t}Bu represents a *tert*-butyl group, and Et represents an ethyl group.

### Example 1: Preparation of 9,9-diethoxy-2-nonanol (9A: R³ = R⁴ = Et)

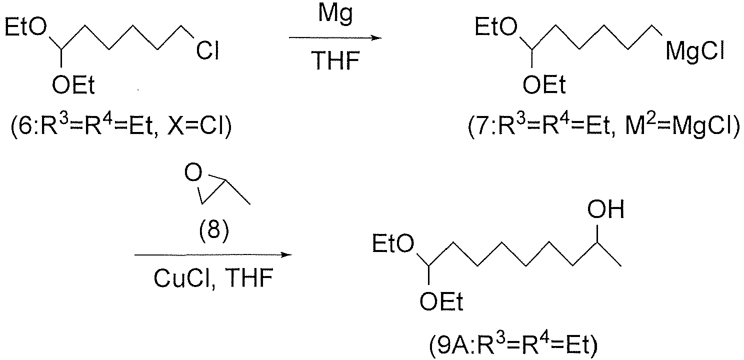

Magnesium (89.30 g, 3.68 gram atoms) and tetrahydrofuran (1,050.00 g) were placed in a reactor at a room temperature and stirred for 42 minutes at 60 to 65°C. 6-Chloro-1,1-diethoxyhexane (6: R³ = R⁴ = Et, X = Cl) (744.93 g, 3.50 mol, purity 98.08%) was then added dropwise to the reactor at 60 to 75°C. After the completion of the dropwise addition, the reaction mixture was stirred for 3 hours at 75 to 80°C to form 6,6-diethoxyhexylmagnesium chloride (7: R³ = R⁴ = Et, M² = MgCl).

The internal temperature of the aforesaid reactor was then cooled to 0 to 10°C, cuprous chloride (0.73 g, 0.0070 mol) was added to the reactor, and then propylene oxide (233.77 g, 4.02 mol) was added dropwise to the reactor at 5 to 30°C. After the completion of the dropwise addition, the reaction mixture was stirred for 2.5 hours at 15 to 25°C. An aqueous solution of acetic acid (prepared from acetic acid (477.65 g) and water (964.31 g)) was then added to the reaction mixture, followed by phase separation. The organic layer thus obtained was concentrated at a reduced pressure, and the resulting concentrate was distilled at a reduced pressure to obtain 9,9-diethoxy-2-nonanol (9A: R³ = R⁴ = Et) (727.52 g, 3.06 mol, purity 97.70%, b.p. = 132.0 to 141.0°C/0.041 kPa (0.31 mmHg)) with a yield of 87.39%.

The following is the spectrum data of 9,9-diethoxy-2-nonanol (9A: R³ = R⁴ = Et) thus prepared.

Nuclear magnetic resonance spectrum: ¹H-NMR (500 MHz, CDCl₃): δ = 1.12-1.23 (9H, m), 1.23-1.62 (13H, m), 3.43-3.52 (2H, m), 3.56-3.65 (2H, m).3.54 (1H, dt, J = 7.2 Hz, 7.2 Hz), 4.45 (1H, t, J = 5.8 Hz); ¹³C-NMR (125 MHz, CDCl₃): δ = 15.30, 18.36, 23.43, 24.63, 25.63, 29.38, 33.52, 39.27, 60.78, 68.03, 102.89.

Mass spectrum: EI-mass spectrum (70 eV): m/z 231 (M⁺-1), 215, 187, 169, 141, 123, 103, 75, 47, 29.

Infrared absorption spectrum: (D-ATR): v = 3421, 2974, 2930, 2858, 1457, 1374, 1345, 1126, 1062, 996, 944 cm⁻¹.

### Example 2: Preparation of 10,10-diethoxy-3-decanol (9B: R³ = R⁴ = Et)

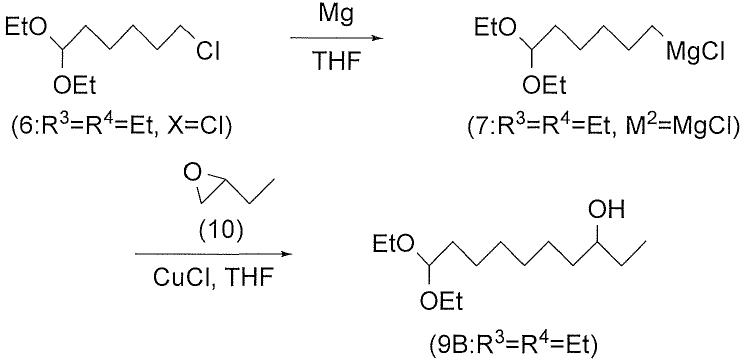

Magnesium (89.30 g, 3.68 gram atoms) and tetrahydrofuran (1,050.00 g) were placed in a reactor at a room temperature and stirred for 37 minutes at 60 to 65°C. 6-Chloro-1,1-diethoxyhexane (6: R³ = R⁴ = Et, X = Cl) (744.93 g, 3.50 mol, purity 98.08%) was then added dropwise to the reactor at 60 to 75°C. After the completion of the dropwise addition, the reaction mixture was stirred for 3 hours at 75 to 80°C to form 6,6-diethoxyhexylmagnesium chloride (7: R³ = R⁴ = Et, M² = MgCl).

The internal temperature of the aforesaid reactor was then cooled to 0 to 10°C, cuprous chloride (0.73 g, 0.0070 mol) was added to the reactor, and then 1,2-butylene oxide (290.24 g, 4.02 mol) was added dropwise to the reactor at 5 to 30°C. After the completion of the dropwise addition, the reaction mixture was stirred for 2.5 hours at 15 to 25°C. An aqueous solution of acetic acid (prepared from acetic acid (477.65 g) and water (964.31 g)) was then added to the reaction mixture, followed by phase separation. The organic layer thus obtained was concentrated at a reduced pressure, and the resulting concentrate was distilled at a reduced pressure to obtain 10,10-diethoxy-3-decanol (9B: R³ = R⁴ = Et) (767.62 g, 3.06 mol, purity 98.21%, b.p. = 140.0 to 145.6°C/0.17 kPa (1.3 mmHg)) with a yield of 87.42%.

The following is the spectrum data of 10,10-diethoxy-3-decanol (9B: R³ = R⁴ = Et) thus prepared.

Nuclear magnetic resonance spectrum: ¹H-NMR (500 MHz, CDCl₃): δ = 0.92 (3H, t, J = 7.2 Hz), 1.18 (6H, t, J = 7.2 Hz), 1.22 (1H, t, J = 7.2 Hz), 1.25-1.62 (14H, m), 3.42-3.53 (2H, m), 3.58-3.65 (2H, m), 3.69 (1H, dt, J = 6.9 Hz, 6.9 Hz), 4.45 (1H, t, J = 5.8 Hz); ¹³C-NMR (125 MHz, CDCl₃): δ = 9.09, 9.82, 15.31, 24.65, 25.52, 29.40, 29.58, 30.10, 33.53, 36.86, 60.78, 102.89.

Mass spectrum: El-mass spectrum (70 eV): m/z 245 (M⁺-1), 229, 201, 183, 171, 155, 137, 103, 75, 47, 29.

Infrared absorption spectrum: (D-ATR): v = 3430, 2974, 2930, 2858, 1462, 1374, 1344, 1126, 1061, 999 cm⁻¹.

### Example 3: Preparation of 9,9-diethoxy-2-acetyloxynonane (5A: R³ = R⁴ = Et, R¹ = CH₃)

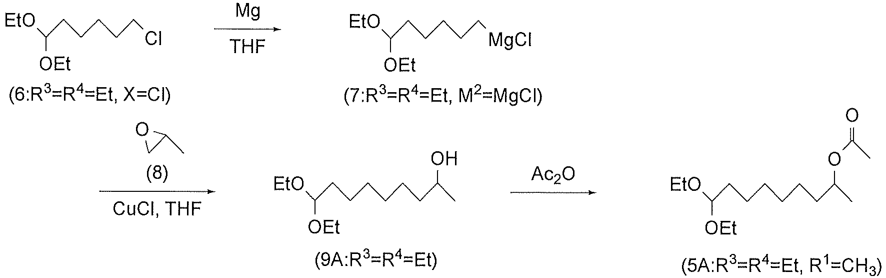

Magnesium (89.30 g, 3.68 gram atoms) and tetrahydrofuran (1,050.00 g) were placed in a reactor at a room temperature and stirred for 20 minutes at 60 to 65°C. 6-Chloro-1,1-diethoxyhexane (6: R³ = R⁴ = Et, X = Cl) (744.93 g, 3.50 mol, purity 98.08%) was then added dropwise to the reactor at 60 to 75°C. After the completion of the dropwise addition, the reaction mixture was stirred for 3 hours at 75 to 80°C to form 6,6-diethoxyhexylmagnesium chloride (7: R³ = R⁴ = Et, M² = MgCl).

The internal temperature of the aforesaid reactor was then cooled to 0 to 10°C, cuprous chloride (0.73 g, 0.0070 mol) was added to the reactor, and then propylene oxide (233.77 g, 4.02 mol) was added dropwise to the reactor at 5 to 30°C. After the completion of the dropwise addition, the reaction mixture was stirred for 2 hours at 15 to 25°C. Acetic anhydride (410.91 g, 4.02 mol) was then added dropwise to the reaction mixture at 20 to 60°C and stirred for 1.5 hours at 30 to 40°C. Water (964.31 g) was then added to the reaction mixture, followed by phase separation. The organic layer thus obtained was concentrated at a reduced pressure, and the resulting concentrate was distilled at a reduced pressure to obtain 9,9-diethoxy-2-acetyloxynonane (5A: R³ = R⁴ = Et, R¹ = CH₃) (874.02 g, 3.11 mol, purity 97.67%, b.p. = 140.0 to 147.9°C/0.44 kPa (3.3 mmHg)) with a yield of 88.89%.

The following is the spectrum data of 9,9-diethoxy-2-acetyloxynonane (5A: R³ = R⁴ = Et, R¹ = CH₃) thus prepared.

Nuclear magnetic resonance spectrum: ¹H-NMR (500 MHz, CDCl₃): δ = 1.16-1.20 (9H, m), 1.22-1.36 (8H, m), 1.39-1.61 (4H, m), 2.00 (3H, s), 3.43-3.50 (2H, m), 3.58-3.65 (2H, m), 4.45 (1H, t, J = 5.7 Hz), 4.86 (1H, tq, J = 6.1 Hz, 6.1 Hz); ¹³C-NMR (125 MHz, CDCl₃): δ = 15.31, 19.90, 21.33, 24.61, 25.26, 29.31, 33.51, 35.84, 60.78, 70.96, 102.87, 170.72.

Mass spectrum: EI-mass spectrum (70 eV): m/z 273 (M⁺-1), 229, 215, 185, 169, 123, 103, 75, 57, 43, 29.

Infrared absorption spectrum: (D-ATR): v = 2975, 2933, 1860, 1738, 1445, 1373, 1347, 1244, 1127, 1062, 953 cm⁻¹.

### Example 4: Preparation of 10,10-diethoxy-3-acetyloxydecane (5B: R³ = R⁴ = Et, R¹ = CH₃)

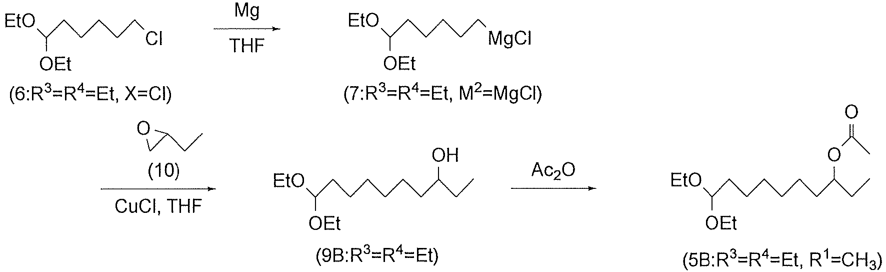

Magnesium (89.30 g, 3.68 gram atoms) and tetrahydrofuran (1,050.00 g) were placed in a reactor at a room temperature and stirred for 20 minutes at 60 to 65°C. 6-Chloro-1,1-diethoxyhexane (6: R³ = R⁴ = Et, X = Cl) (744.93 g, 3.50 mol, purity 98.08%) was then added dropwise to the reactor at 60 to 75°C. After the completion of the dropwise addition, the reaction mixture was stirred for 3 hours at 75 to 80°C to form 6,6-diethoxyhexylmagnesium chloride (7: R³ = R⁴ = Et, M² = MgCl).

The internal temperature of the aforesaid reactor was then cooled to 0 to 10°C, cuprous chloride (0.73 g, 0.0070 mol) was added to the reactor, and then 1,2-butylene oxide (290.24 g, 4.02 mol) was added dropwise to the reactor at 5 to 30°C. After the completion of the dropwise addition, the reaction mixture was stirred for 2 hours at 15 to 25°C. Acetic anhydride (410.91 g, 4.02 mol) was then added dropwise to the reaction mixture at 20 to 60°C and stirred for 1.5 hours at 30 to 40°C. Water (964.31 g) was then added to the reaction mixture, followed by phase separation. The organic layer thus obtained was concentrated at a reduced pressure, and the resulting concentrate was distilled at a reduced pressure to obtain 10,10-diethoxy-3-acetyloxydecane (5B: R³ = R⁴ = Et, R¹ = CH₃) (897.83, 3.03 mol, purity 97.24%, b.p. = 143.0 to 148.1°C/0.059 kPa (0.44 mmHg)) with a yield of 86.50%.

The following is the spectrum data of 10,10-diethoxy-3-acetyloxydecane (5B: R³ = R⁴ = Et, R¹ = CH₃) thus prepared.

Nuclear magnetic resonance spectrum: ¹H-NMR (500 MHz, CDCl₃): δ = 0.86 (3H, t, J = 7.3 Hz), 1.18 (6H, t, J = 7.3 Hz), 1.21-1.37 (8H, m), 1.45-1.62 (6H, m), 2.02 (3H, s), 3.43-3.51 (2H, m), 3.58-3.66 (2H, m), 4.45 (1H, t, J = 5.7 Hz), 4.78 (1H, tt, J = 6.3 Hz, 6.3 Hz); ¹³C-NMR (125 MHz, CDCl₃): δ = 9.53, 15.31, 21.20, 24.62, 25.19, 26.89, 29.32, 29.41, 33.51, 60.78, 75.45, 102.88, 170.91.

Mass spectrum: EI-mass spectrum (70 eV): m/z 287 (M⁺-1), 243, 229, 199, 183, 137, 103, 75, 57, 43, 29.

Infrared absorption spectrum: (D-ATR): v = 2973, 2933, 2860, 1738, 1461, 1373, 1243, 1127, 1063, 1019, 955 cm⁻¹.

### Example 5: Preparation of 8-(acetyloxy)nonanal (1: R¹ = CH₃, n = 0)

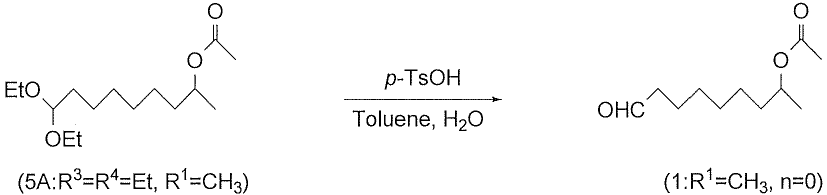

The 9,9-diethoxy-2-acetyloxynonane (5A: R³ = R⁴ = Et, R¹ = CH₃) (103.58, 0.37 mol, purity 97.67%) obtained in Example 3,p-toluenesulfonic acid monohydrate (0.50 g, 0.0026 mol), water (66.40 g, 3.69 mol), and toluene (40.00 g) were placed in a reactor at a room temperature, and ethanol was distilled off at 60 to 70°C. After the conversion was confirmed to be 99% by GC, the reaction mixture was then cooled to 20 to 30°C, and the aqueous layer was removed to obtain the organic layer. The organic layer thus obtained was distilled at a reduced pressure to obtain 8-(acetyloxy)nonanal (1: R¹ = CH₃, n = 0) (67.51 g, 0.33 mol, purity 98.85%, b.p. = 110.0 to 118.1°C/0.19 kPa (1.4 mmHg)) with a yield of 90.38%.

The following is the spectrum data of 8-(acetyloxy)nonanal (1: R¹ = CH₃, n = 0) thus prepared.

Nuclear magnetic resonance spectrum: ¹H-NMR (500 MHz, CDCl₃): δ = 1.17 (3H, d, J = 6.5 Hz), 1.22-1.35 (6H, m), 1.40-1.49 (1H, m), 1.50-1.65 (3H, m), 2.01 (3H, s), 2.40 (2H, dt, J = 1.9 Hz, 7.3 Hz), 4.86 (1H, tq, J = 6.5 Hz, 6.5 Hz), 9.74 (1H, t, J = 1.9 Hz); ¹³C-NMR (125 MHz, CDCl₃): δ = 19.89, 21.32, 21.90, 25.11, 28.96, 29.09, 35.75, 43.79, 70.84, 170.72, 202.70.

Mass spectrum: EI-mass spectrum (70 eV): m/z 201 (M⁺+1), 157, 140, 122, 112, 97, 81, 67, 55, 43, 29.

Infrared absorption spectrum: (D-ATR): v = 2977, 2934, 2859, 2719, 1732, 1462, 1373, 1245, 1137, 1022, 952 cm⁻¹.

### Example 6: Preparation of 8-(acetyloxy)decanal (1: R¹ = CH₃, n = 1)

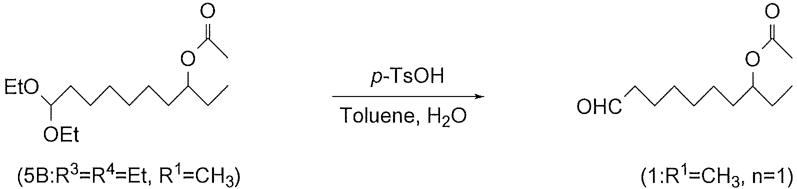

The 10,10-diethoxy-3-acetyloxydecane (5B: R³ = R⁴ = Et, R¹ = CH₃) (109.36 g, 0.37 mol, purity 97.24%) obtained in Example 4, p-toluenesulfonic acid monohydrate (0.50 g, 0.0026 mol), water (66.40 g, 3.69 mol), and toluene (40.00 g) were placed in a reactor at a room temperature, and ethanol was distilled off at 60 to 70°C. After the conversion was confirmed to be 99% by GC, the reaction mixture was then cooled to 20 to 30°C, and the aqueous layer was removed to obtain the organic layer. The organic layer thus obtained was distilled at a reduced pressure to obtain 8-(acetyloxy)decanal (1: R¹ = CH₃, n = 1) (67.45 g, 0.31 mol, purity 98.11%, b.p. = 110.0 to 119.1°C/0.19 kPa (1.4 mmHg)) with a yield of 83.75%.

The following is the spectrum data of 8-(acetyloxy)decanal (1: R¹ = CH₃, n = 1) thus prepared.

Nuclear magnetic resonance spectrum: ¹H-NMR (500 MHz, CDCl₃): δ = 0.85 (3H, t, J = 7.3 Hz), 1.20-1.35 (6H, m), 1.45-1.66 (6H, m), 2.02 (3H, s), 2.40 (2H, dt, J = 1.9 Hz, 7.3 Hz), 4.77 (1H, quint-like, J = 6.1 Hz), 9.74 (1H, t, J = 1.9 Hz); ¹³C-NMR (125 MHz, CDCl₃): δ = 9.52, 21.18, 21.90, 25.04, 26.89, 28.97, 29.16, 33.44, 43.78, 75.31, 170.91, 202.70.

Mass spectrum: El-mass spectrum (70 eV): m/z 215 (M⁺+1), 185, 171, 156, 143, 125, 113, 101, 81, 69, 55, 43, 29.

Infrared absorption spectrum: (D-ATR): v = 2968, 2935, 2859, 2719, 1732, 1463, 1373, 1245, 1113, 1020, 956 cm⁻¹.

### Example 7: Preparation of 2,9-diacetoxyundecane (4)

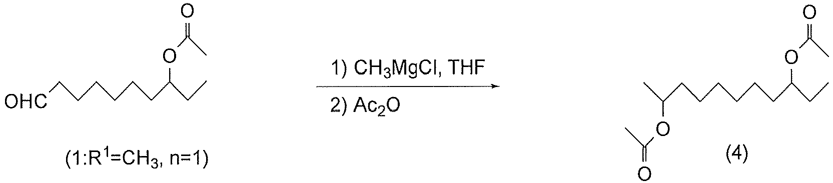

A tetrahydrofuran solution (399.35 g) of methylmagnesium chloride (with 1.03 mol of methylmagnesium chloride) was placed in a reactor at 15 to 25°C, and then the 8-(acetyloxy)decanal (1: R¹ = CH₃, *n* = 1) (67.45 g, 0.31 mol, purity 98.11%) obtained in Example 6 was added dropwise at 40 to 60°C and stirred for 4 hours at 30 to 40°C. Acetic anhydride (111.73 g, 1.09 mol) was then added dropwise at 40 to 60°C and stirred for 2 hours at 30 to 40°C. Water (500.00 g) was then added to the reaction mixture, followed by phase separation. The aqueous layer was removed to obtain the organic layer. The organic layer thus obtained was concentrated at a reduced pressure, and the resulting concentrate was distilled at a reduced pressure to obtain 2,9-diacetoxyundecane (4) (80.73 g, 0.28 mol, purity 95.01%, b.p. = 115.6 to 122.1°C/0.080 kPa (0.60 mmHg)) with a yield of 90.04% in two steps.

The following is the spectrum data of 2,9-diacetoxyundecane (4) thus prepared.

Nuclear magnetic resonance spectrum: ¹H-NMR (500 MHz, CDCl₃): δ = 0.86 (3H, t, J = 7.3 Hz), 1.18 (3H, d, J = 6.5 Hz), 1.20-1.33 (8H, m), 1.37-1.60 (6H, m), 2.00 (3H, s), 2.02 (3H, s), 4.78 (1H, quint-like, J = 6.6 Hz), 4.86 (1H, tq, J = 6.5 Hz, 6.5 Hz); ¹³C-NMR (125 MHz, CDCl₃): δ = 9.53, 19.91, 21.20, 21.33, 25.18, 25.25, 26.90, 29.26, 29.34, 33.50, 35.82, 70.93, 75.40, 170.72, 170.92.

Mass spectrum: EI-mass spectrum (70 eV): m/z 273 (M⁺+1), 229, 215, 201, 183, 169, 155, 141, 123, 96, 81, 67, 43, 29.

Infrared absorption spectrum: (D-ATR): vmax = 2971, 2935, 2859, 1737, 1463, 1372, 1244, 1128, 1021, 953 cm⁻¹.

### Example 8: Preparation of 2,9-diacetoxyundecane (4)

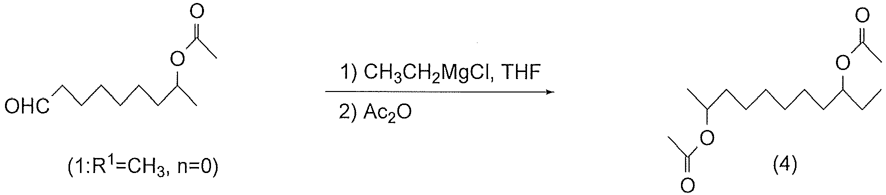

A tetrahydrofuran solution (816.59 g) of ethylmagnesium chloride (with 1.67 mol of methylmagnesium chloride) was placed in a reactor at 15 to 25°C, and then 8-(acetyloxy)nonanal (1: R¹ = CH₃, n = 0) (103.32 g, 0.51 mol, purity 98.08%) prepared as in Example 5 was added dropwise at 40 to 60°C and stirred for 5 hours at 30 to 40°C. Acetic anhydride (180.80 g, 1.77 mol) was then added dropwise at 40 to 60°C and stirred for 3.5 hours at 30 to 40°C. Water (809.14 g) was then added to the reaction mixture, followed by phase separation. The aqueous layer was removed to obtain the organic layer. The organic layer thus obtained was concentrated at a reduced pressure, and the resulting concentrate was distilled at a reduced pressure to obtain 2,9-diacetoxyundecane (4) (122.60 g, 0.42 mol, purity 92.38%, b.p. = 115.6 to 122.1°C/0.080 kPa (0.60 mmHg)) with a yield of 82.18% in two steps.

The spectrum data of 2,9-diacetoxyundecane (4) thus obtained was the same as that obtained in Example 7.

### Example 9: Preparation of 8-(trimethylacetyloxy)decanal (1: R¹ = C(CH₃)₃, n = 1)

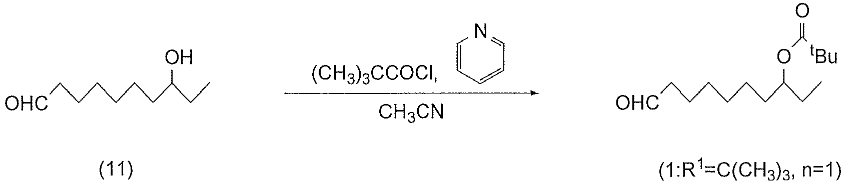

8-Hydroxydecanal (11) (4.0 g, 0.023 mol, purity 98.59%) prepared as in Comparative Example 1 below, pyridine (2.90 g, 0.037 mol), and acetonitrile (10.00 g) were placed in a reactor at a room temperature and stirred for 14 minutes at 15 to 25°C. Pivaloyl chloride (2.76 g, 0.023 mol) was then added dropwise at 20 to 30°C and stirred for 8 hours at 20 to 30°C. Water (15.00 g) was then added to the reaction mixture, followed by phase separation. The aqueous layer was removed to obtain the organic layer. The organic layer thus obtained was concentrated at a reduced pressure, and the resulting concentrate was purified by silica gel column chromatography (hexane:ethyl acetate = 30:1 to 5:1) to obtain 8-(trimethylacetyloxy)decanal (1: R¹ = C(CH₃)₃, *n* = 1) (2.08 g, 0.0080 mol, purity 98.24%) with a yield of 34.89%.

The following is the spectrum data of 8-(trimethylacetyloxy)decanal (1: R¹ = C(CH₃)₃, *n* = 1) thus prepared.

Nuclear magnetic resonance spectrum: ¹H-NMR (500 MHz, CDCl₃): δ = 0.85 (3H, t, J = 7.3 Hz), 1.18 (9H, s), 1.21-1.34 (6H, m), 1.43-1.64 (6H, m), 2.40 (2H, dt, J = 1.9 Hz, 7.3 Hz), 4.76 (1H, tt, J = 7.3 Hz, 5.3 Hz), 9.74 (1H, t, J = 1.9 Hz); ¹³C-NMR (125 MHz, CDCl₃): δ = 9.48, 21.92, 25.01, 26.93, 27.18, 28.99, 29.17, 33.50, 38.82, 43.80, 74.75, 178.26, 202.72.

Mass spectrum: EI-mass spectrum (70 eV): m/z 257 (M⁺+1), 227,213,199,171, 155, 137, 95, 81, 57, 41.

Infrared absorption spectrum: (D-ATR): vmax = 2970, 2935, 2860, 2716, 1724, 1480, 1462, 1396, 1367, 1284, 1166, 1113, 1032, 936 cm⁻¹.

### Example 10: Preparation of 8-(benzoyloxy)decanal (1: R¹ = Ph, n = 1)

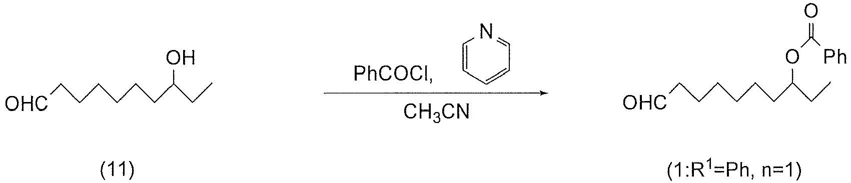

8-Hydroxydecanal (11) (4.0 g, 0.023 mol, purity 98.59%) prepared as in Comparative Example 1 below, pyridine (2.90 g, 0.037 mol), and acetonitrile (10.00 g) were placed in a reactor at a room temperature and stirred for 10 minutes at 15 to 25°C. Benzoyl chloride (3.22 g, 0.023 mol) was then added dropwise at 20 to 30°C and stirred for 7 hours at 20 to 30°C. Water (15.00 g) was then added to the reaction mixture, followed by phase separation. The aqueous layer was removed to obtain the organic layer. The organic layer thus obtained was concentrated at a reduced pressure, and the resulting concentrate was purified by silica gel column chromatography (hexane:ethyl acetate = 10:1 to 5:1) to obtain 8-(benzoyloxy)decanal (1: R¹ = Ph, n = 1) (1.13 g, 0.0041 mol, purity 100%) with a yield of 17.80%.

The following is the spectrum data of 8-(benzoyloxy)decanal (1: R¹ = Ph, n = 1) thus prepared.

Nuclear magnetic resonance spectrum: ¹H-NMR (500 MHz, CDCl₃): δ = 0.94 (3H, t, J = 7.3 Hz), 1.26-1.44 (6H, m), 1, 54-1.76 (6H, m), 2.39 (2H, dt, J = 1.9 Hz, 7.3 Hz), 5.03-5.10 (1H, m), 7.43 (2H, t-like, J = 8.1 Hz), 7.52-7.57 (1H, m), 8.02-8.06 (2H, m), 9.73 (1H, t, J = 1.9 Hz); ¹³C-NMR (125 MHz, CDCl₃): δ = 9.61, 21.92, 25.10, 27.06, 29.97, 29.19, 33.56, 43.78, 75.98, 128.27, 129.48, 130.76, 132.68, 166.36, 202.72.

Mass spectrum: EI-mass spectrum (70 eV): m/z 275 (M⁺-1), 248, 233, 218, 200, 154, 123, 105, 77, 55, 41.

Infrared absorption spectrum: (D-ATR): v = 2968, 2935, 2858, 2718, 1716, 1602, 1585, 1451, 1275, 1176, 1107, 1070, 1026 cm⁻¹.

### Comparative Example 1: Preparation of 8-hydroxynonanal (11)

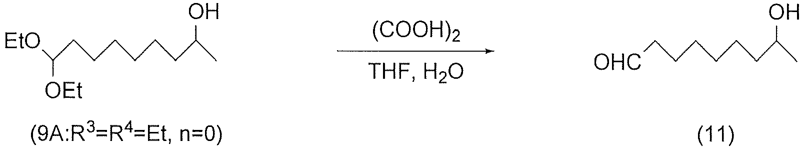

9,9-Diethoxy-2-nonanol (9A: R³ = R⁴ = Et, *n* = 0) (200.00 g, 0.84 mol, purity 97.70%) prepared in Example 1, oxalic acid dihydrate (318.04 g, 2.52 mol), tetrahydrofuran (840.90 g), and water (840.90 g) were placed in a reactor at a room temperature and stirred for 2.5 hours at 60 to 65°C. Hexane (494.62 g) was then added and stirred for 30 minutes. After the completion of the stirring, the reaction mixture was left to stand for phase separation, followed by removal of the aqueous layer to obtain the organic layer. The organic layer thus obtained was concentrated at a reduced pressure, and the resulting concentrate was distilled at a reduced pressure to obtain 8-hydroxynonanal (11) (18.29 g, 0.10 mol, purity 88.26%) with a yield of 12.12%. The yield was extremely low compared to the process for preparing the 8-(acyloxy)alkanal compound (1).

The following is the spectrum data of 8-hydroxynonanal (11) thus prepared.

Nuclear magnetic resonance spectrum: ¹H-NMR (500 MHz, CDCl₃): δ = 1.15 (3H, d, J = 6.1 Hz), 1.20-1.49 (8H, m), 1.56-1.65 (2H, m), 1.71 (1H, br.s), 2.40 (2H, dt, J = 1.9 Hz, 7.3 Hz), 3.71-3.80 (1H, m), 9.73 (1H, t, J = 1.9 Hz); ¹³C-NMR (125 MHz, CDCl₃): δ = 21.90, 23.42, 25.45, 29.02, 29.26, 39.11, 43.78, 67.93, 202.83.

Mass spectrum: EI-mass spectrum (70 eV): m/z 157 (M⁺-1), 143, 125, 114, 96, 81, 68, 57, 45, 29.

Infrared absorption spectrum: (D-ATR): v = 3406, 2930, 2857, 1724, 1463, 1373, 1112, 1062, 943 cm⁻¹.

## Claims

1. An 8-(acyloxy)alkanal compound of the following general formula (1): 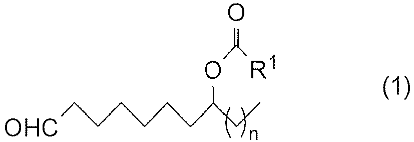 wherein n represents 0 or 1, and R¹ represents a linear or branched alkyl group having 1 to 10 carbon atoms, a phenyl group, or a phenyl group in which one or more hydrogen atoms are substituted with a halogen atom.

2. A process for preparing 2,9-diacetoxyundecane of the following formula (4): 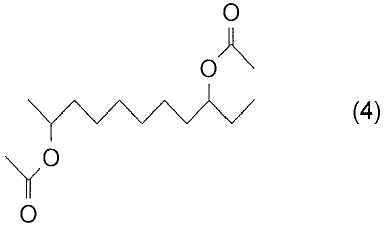 the process comprising the steps of
subjecting an 8-(acyloxy)alkanal compound of the following general formula (1): 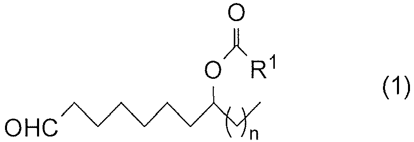 wherein n represents 0 or 1, and R¹ represents a linear or branched alkyl group having 1 to 10 carbon atoms, a phenyl group, or a phenyl group in which one or more hydrogen atoms are substituted with a halogen atom,
to a nucleophilic addition reaction with a nucleophilic reagent, alkyl compound, of the following general formula (2):
CH₃(CH₂)_{(1-*n*})M¹ (2)
wherein M¹ represents Li, MgZ¹, CuZ¹, or CuLiZ¹, n represents 0 or 1, and Z¹ represents a halogen atom, a methyl group, or an ethyl group,
to form 2,9-undecanediol of the following formula (3): 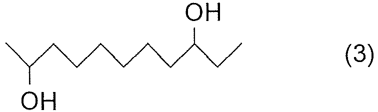 and subjecting the 2,9-undecanediol (3) to an acetylation reaction to form the aforesaid 2,9-diacetoxyundecane (4).

3. A process for preparing an 8-(acyloxy)alkanal compound of the following general formula (1): 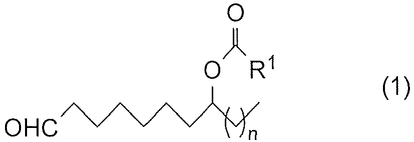
wherein n represents 0 or 1, and R¹ represents a linear or branched alkyl group having 1 to 10 carbon atoms, a phenyl group, or a phenyl group in which one or more hydrogen atoms are substituted with a halogen atom,
the process comprising the step of
subjecting a dialkoxyacyloxyalkane compound of the following general formula (5): 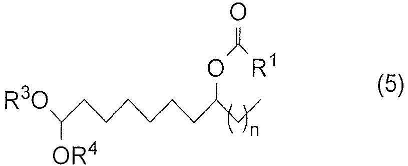 wherein n represents 0 or 1, R¹ is as defined above, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
to a hydrolysis reaction to form the aforesaid 8-(acyloxy)alkanal compound (1).

4. A process for preparing the 8-(acyloxy)alkanal compound (1) as defined in claim 3, with proviso that n is 0,
the process comprising the steps of
converting a 6-halo-1,1-dialkoxyhexane compound of the following general formula (6): 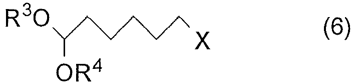 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
into a nucleophilic reagent, 6,6-dialkoxyhexyl compound, of the following general formula (7): 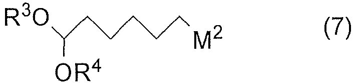 wherein M² represents Li, MgZ², CuZ², or CuLiZ², Z² represents a halogen atom or a 6,6-dialkoxyhexyl group, and R³ and R⁴ are as defined above,
subsequently subjecting the nucleophilic reagent, 6,6-dialkoxyhexyl compound (7), to a nucleophilic addition reaction with propylene oxide of the following formula (8): 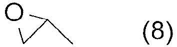 to form a 9,9-dialkoxy-2-nonanol compound of the following general formula (9A): 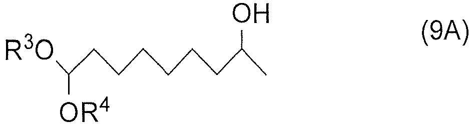 wherein R³ and R⁴ are as defined above,
subjecting the 9,9-dialkoxy-2-nonanol compound (9A) to acylation to form the dialkoxyacyloxyalkane compound (5) as defined in claim 3 with proviso that n is 0;
and the process according to claim 3 for preparing the 8-(acyloxy)alkanal compound (1) as defined in claim 3, with proviso that n is 0, by subjecting the aforesaid dialkoxyacyloxyalkane compound (5) as defined in claim 3 with proviso that n is 0, to a hydrolysis reaction.

5. A process for preparing the 8-(acyloxy)alkanal compound (1) as defined in claim 3, with proviso that *n* is 1,
the process comprising the steps of
converting a 6-halo-1,1-dialkoxyhexane compound of the following general formula (6): 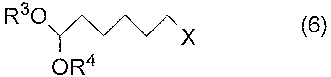 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
into a nucleophilic reagent, 6,6-dialkoxyhexyl compound, of the following general formula (7): 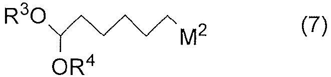 wherein M² represents Li, MgZ², CuZ², or CuLiZ², Z² represents a halogen atom or a 6,6-dialkoxyhexyl group, and R³ and R⁴ are as defined above,
subsequently subjecting the nucleophilic reagent, 6,6-dialkoxyhexyl compound (7), to a nucleophilic addition reaction with 1,2-butylene oxide of the following formula (10): 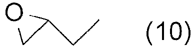 to form a 10,10-dialkoxy-3-decanol compound of the following general formula (9B): 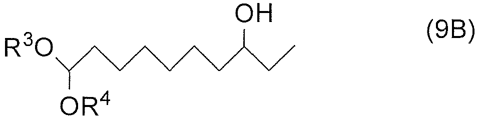 wherein R³ and R⁴ are as defined above,
subjecting the 10,10-dialkoxy-3-decanol (9B) to acylation to form the dialkoxyacyloxyalkane compound (5) as defined in claim 3 with proviso that n is 1;
and the process according to claim 3 for preparing the 8-(acyloxy)alkanal compound (1) as defined in claim 3, with proviso that *n* = 1, by subjecting the dialkoxyacyloxyalkane compound (5) as defined in claim 3 with proviso that n is 1 to a hydrolysis reaction.

6. A process for preparing 2,9-diacetoxyundecane (4) as defined in claim 2,
the process comprising steps of
the process for preparing the aforesaid 8-(acyloxy)alkanal compound (1) from the aforesaid dialkoxyacyloxyalkane compound (5), according to claim 3, and
the process for preparing the aforesaid 2,9-diacetoxyundecane (4) from the aforesaid 8-(acyloxy)alkanal compound (1), according to claim 2.

7. A process for preparing 2,9-diacetoxyundecane (4) as defined in claim 2,
the process comprising the steps of
converting a 6-halo-1,1-dialkoxyhexane compound of the following general formula (6): 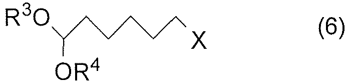 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
into a nucleophilic reagent, 6,6-dialkoxyhexyl compound, of the following general formula (7): 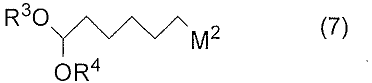 wherein M² represents Li, MgZ², CuZ², or CuLiZ², Z² represents a halogen atom or a 6,6-dialkoxyhexyl group, and R³ and R⁴ are as defined above,
subsequently subjecting the nucleophilic reagent, 6,6-dialkoxyhexyl compound (7), to a nucleophilic addition reaction with propylene oxide of the following formula (8): 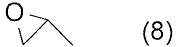 to form a 9,9-dialkoxy-2-nonanol compound of the following general formula (9A): 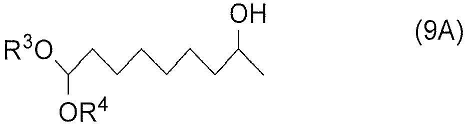 wherein R³ and R⁴ are as defined above,
subjecting the 9,9-dialkoxy-2-nonanol compound (9A) to acylation to form a 9,9-dialkoxy-2-acyloxynonane compound of the following general formula (5A): 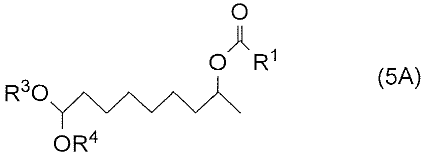 wherein R¹, R³, and R⁴ are as defined above,
subjecting the 9,9-dialkoxy-2-acyloxynonane compound (5A) to a hydrolysis reaction to form an 8-(acyloxy)alkanal compound (1) as defined in claim 2, with proviso that *n* = 0,
and the process for preparing the 2,9-diacetoxyundecane (4) from the 8-(acyloxy)alkanal compound (1) as defined in claim 3 with proviso that n = 0, according to claim 2.

8. A process for preparing 2,9-diacetoxyundecane (4) as defined in claim 2,
the process comprising the steps of
converting a 6-halo-1,1-dialkoxyhexane compound of the following general formula (6): 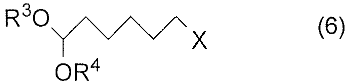 wherein X represents a halogen atom, and R³ and R⁴ represent, independently of each other, a monovalent hydrocarbon group having 1 to 15 carbon atoms; or R³ and R⁴ form together a divalent hydrocarbon group, R³-R⁴, having 2 to 10 carbon atoms,
into a nucleophilic reagent, 6,6-dialkoxyhexyl compound, of the following general formula (7): 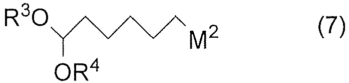 wherein M² represents Li, MgZ², CuZ², or CuLiZ², Z² represents a halogen atom or a 6,6-dialkoxyhexyl group, and R³ and R⁴ are as defined above,
subsequently subjecting the nucleophilic reagent, 6,6-dialkoxyhexyl compound (7), to a nucleophilic addition reaction with 1,2-butylene oxide of the following formula (10): 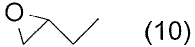 to form a 10,10-dialkoxy-3-decanol compound of the following general formula (9B): 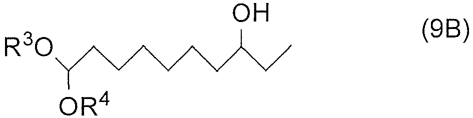 wherein R³ and R⁴ are as defined above,
subjecting the 10,10-dialkoxy-3-decanol compound (9B) to acylation to form a 10,10-dialkoxy-3-acyloxy decane compound of the following general formula (5B): 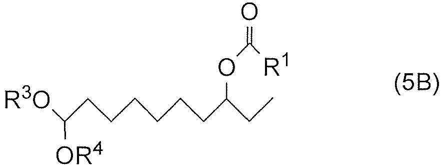 wherein R¹, R³, and R⁴ are as defined above,
subjecting the 10,10-dialkoxy-3-acyloxydecane compound (5B) to a hydrolysis reaction to form the 8-(acyloxy)alkanal compound (1) as defined in claim 2, with proviso that n = 1,
and the process for preparing the 2,9-diacetoxyundecane (4) from the 8-(acyloxy)alkanal compound (1) as defined in claim 3, with proviso that wherein n = 1, according to claim 2.
